# EUROPÄISCHE PATENTSCHRIFT

(11) **EP 3 372 183 B1**
(45) Veröffentlichungstag und Bekanntmachung des Hinweises auf die Patenterteilung: **04.09.2019**
(21) Anmeldenummer: 17160265.9
(22) Anmeldetag: 10.03.2017
(51) Int. Cl.: A61B 18/04, A61B 18/14

(54) **INSTRUMENT ZUR ABLATION**
INSTRUMENT FOR ABLATION
INSTRUMENT D'ABLATION

(43) Veröffentlichungstag der Anmeldung: 12.09.2018
(73) Patentinhaber: Erbe Elektromedizin GmbH, 72072 Tübingen (DE)
(72) Erfinder: Stäbler, Thomas, 72070 Tübingen (DE); Herrberg, Charlotte, 72411 Bodelshausen (DE); Brodbeck, Achim, 72555 Metzingen (DE)
(74) Vertreter: Rüger Abel

(56) Entgegenhaltungen:
- EP-A1- 0 346 613
- WO-A1-2008/090004
- US-A- 4 730 936
- US-A1- 2005 118 350
- US-A1- 2006 184 097
- US-A1- 2011 184 408

## Beschreibung

Die Erfindung betrifft eine Vorrichtung zur Ablation, insbesondere für die Mucosaablation.

WO 2008/090004 A1 beschreibt ein bipolares Instrument zur Behandlung von Gewebe mit zwei Kanälen, in denen jeweils eine Elektrode angeordnet ist. Die Kanäle dienen dem Zuführen von inertem Gas an die Elektroden, um zwischen den beiden Elektroden einen Lichtbogen auszubilden.

Das in US 2011/0184408 A1 dargestellte Instrument zur Erzeugung eines Plasmas zur elektrochirurgischen Behandlung weist zwei Gaskanäle mit jeweils einer Elektrode auf, mit deren Anordnung sich vor dem Instrument vereinende Plasmaentladungen erzeugt werden sollen.

Die therapeutische Mucosaablation, zum Beispiel zur Tumorablation oder zur Zerstörung von das Essverhalten beeinflussenden Zellen der Magenwand vorteilhafterweise zur Gewichtsreduktion, wird typischerweise durch endoskopische Intervention durchgeführt, wobei zur Ablation spezielle Sonden zur Anwendung kommen können.

Beispielsweise ist dazu aus der WO 2011/022069 A2 ein Endoskop mit einer Endkappe bekannt, die auf die Mucosa aufzusetzen ist, und in deren Innenraum eine Argonplasmakoagulation durchgeführt wird. Die Kappe soll den Einwirkungsbereich der Argonplasmakoagulation begrenzen und somit die Mucosakoagulation definieren.

Aus der US 8 641 711 B2 ist ein Instrument zur Abtragung von Gewebeschichten von Hohlorganen bekannt, wobei das Instrument einen elektrisch aktiven Kopf mit Elektroden aufweist, an dem ein expandierbares Element vorgesehen ist, um den Kopf des Instruments in Bezug auf die gegenüberliegende Gewebewand definiert zu positionieren. Dieses Instrument setzt Hohlorgane mit beschränktem Durchmesser voraus, wie es z.B. im Darm der Fall ist.

Die großflächige Mucosaablation stellt an den Behandler insbesondere Herausforderungen hinsichtlich Geduld und Fingerfertigkeit. Dies insbesondere, wenn dazu für den allgemeinen Gebrauch vorgesehene flexible Instrumente, wie Polypektomieschlingen oder dergleichen eingesetzt werden. Mit solchen Schlingen wird in einem Arbeitsschritt lediglich eine Resektion der Mucosa von ca. 2 cm² ausgeführt. Im Fundus- und Cardiabereich des Magens ist eine Resektion mithilfe eines flexiblen Endoskops sehr schwierig. Außerdem besteht das Risiko der Perforation.

Bei einer Ablation der Mucosa ist eine nicht ausreichende Ablation ebenso abzulehnen wie eine zu tiefe Einwirkung, weil dadurch darunter liegende Gewebeschichten beschädigt werden könnten bis hin zur Perforation des Magens. Insgesamt ist eine zügige und sichere Durchführung der Behandlung wünschenswert. Zudem ist eine unerwünschte thermische Belastung des Gewebes im gesamten Behandlungsverlauf zu vermeiden.

Die der Erfindung zugrunde liegende Aufgabe wird mit dem Instrument nach Anspruch 1 gelöst.

Das erfindungsgemäße Instrument ist zur großflächigen Gewebebehandlung, vorzugsweise zur Gewebeablation, beispielsweise zur Mucosaablation, geeignet. Das erfindungsgemäße Instrument weist wenigstens eine erste Gaszuführungsleitung und eine zweite Gaszuführungsleitung auf, wobei die Gaszuführungsleitungen der Zuführung eines Gases, bevorzugt einem Inertgas, insbesondere Argon, dienen. Eine erste Elektrode ist wenigstens teilweise in der ersten Gaszuführungsleitung angeordnet. Eine zweite Elektrode ist wenigstens teilweise in der zweiten Gaszuführungsleitung angeordnet. Die erste Gaszuführungsleitung und die zweite Gaszuführungsleitung sind relativ zueinander derart angeordnet, dass das distale Ende der ersten Gaszuführungsleitung mit dem distalen Ende der zweiten Gaszuführungsleitung einen spitzen Winkel einschließt, so dass die distalen Enden der Gaszuführungsleitungen in distaler Richtung divergieren. Auf Grund des spitzen Winkels können bei Beaufschlagung der Gaszuführungsleitungen mit Gas in dem Ende der ersten Gaszuführungsleitung eine Gasströmung und in dem Ende der zweiten Gaszuführungsleitung eine Gasströmung erzeugt werden, wobei die Strömungsrichtungen der beiden Gasströmungen den spitzen Winkel einschließen. Der Winkel beträgt vorzugsweise zwischen mindestens 1° und maximal 60°, besonders bevorzugt zwischen mindestens 1° und maximal 30°, beispielsweise 10°. Die erste Elektrode und die zweite Elektrode können miteinander ebenfalls einen spitzen Winkel, beispielsweise von demselben Betrag, einschließen, so dass die erste Elektrode und die zweite Elektrode in distaler Richtung divergieren. Die erste Gaszuführungsleitung und die zweite Gaszuführungsleitung sind zueinander derart angeordnet, dass zwischen dem distalen Ende der ersten Gaszuführungsleitung und dem distalen Ende der zweiten Gaszuführungsleitung ein Freiraum angeordnet ist. Dieser erlaubt einen Blick zwischen den Enden der Gaszuführungsleitungen hindurch auf den Einwirkungsbereich des Instruments vor den distalen Enden der Gaszuführungsleitungen.

Wenn die Elektroden mit einer HF-Spannung versorgt werden, kann mittels an den Elektroden erzeugten Funken ein Plasma, beispielsweise ein Argon-Plasma erzeugt werden. Das Plasma kann zu einer Behandlung des vor den Elektroden liegenden Gewebes herangezogen werden. Die Behandlung umfasst insbesondere schneiden und/oder koagulieren. Durch die Anordnung der Enden der Gaszuführungsleitungen zueinander im spitzen Winkel und/oder durch die Anordnung der Elektroden zueinander im spitzen Winkel kann erreicht werden, dass die von den beiden Enden der Gaszuführungsleitungen ausgehenden Plasmaströme divergieren und einen breiten streifenförmigen Bereich in der Mucosa schneiden und/oder koagulieren, wenn das Instrument entlang der Mucosa bewegt wird. Die aus den beiden Enden der Gaszuführungsleitungen austretenden Plasmaströme können gemeinsam einen etwa fächerförmigen Strom bilden, d.h. einen Strahl mit nichtkreisförmigem (ovalem oder streifenartigem) Querschnitt. Die Austrittsöffnungen der beiden Gaszuführungsleitungen liegen vorzugsweise auf einer Linie, die quer zur Bewegungsrichtung des Instruments orientiert ist. Bei geeigneter Festlegung des Winkels zwischen den beiden Enden der Gaszuführungsleitungen und/oder zwischen den beiden Elektroden sowie der Ausströmgeschwindigkeit des Gases aus den Gaszuführungsleitungen, der Gasmenge sowie der elektrischen Beaufschlagung der beiden Elektroden, kann erreicht werden, dass sich über die Breite des koagulierten Streifens der Mucosa eine einheitlichere, im therapeutischen Sinne einheitliche Wirkungstiefe ergibt. Damit lässt sich eine gleichmäßige und großflächige Gewebebehandlung, insbesondere -ablation, erreichen.

Das erfindungsgemäße Konzept ebnet den Weg zu der Gestaltung eines besonders schmalen, schlanken Instrumentenendes das kaum breiter oder im Einzelfall auch schmaler sein kann als die von ihm erzeuge Spur koagulierten Mucosagewebes.

Durch den Freiraum zwischen den Enden der Gaszuführungsleitungen hat der Benutzer des Instruments eine verbesserte Sicht auf den Einwirkungsbereich des Instruments vor den distalen Enden der Gaszuführungsleitungen bzw. vor den Enden der Elektroden. Mit dem Freiraum ist dem Verwender des Instruments vorzugsweise die Möglichkeit gegeben, bei der plasmachirurgischen Behandlung von Gewebe mit dem Instrument schräg zur Längsachse des distalen Endes des Instruments zwischen den distalen Enden der Gaszuführungsleitungen hindurch durch den Freiraum auf den Einwirkungsbereich des Instruments blicken zu können.

Zudem ist durch den Freiraum die Wärmekapazität des Instruments an dem distalen Ende des Instruments relativ gering. Wenn das Ende des Instruments durch die Plasmabehandlung heiß wird, kann ein schnelleres Abkühlen des Endes des Instruments erreicht werden, so dass das Instrument relativ kurze Zeit nach der Plasmabehandlung aus dem Körper des Patienten entfernt werden kann, ohne dass die Gefahr einer Verletzung gesunden Gewebes durch ein heißes Ende des Instruments besteht. Durch das in dem Freiraum "fehlende" Material kommt es dort zu keiner Gewebeanhaftung auf Grund heißen Materials.

Das Verbindungselement für das erfindungsgemäße Instrument weist einen Sitz für die erste Gaszuführungsleitung und einen Sitz für die zweite Gaszuführungsleitung auf. Die Sitze sind derart ausgebildet, dass die Gaszuführungsleitungen in den Sitzen so angeordnet werden können, dass die distalen Enden der Gaszuführungsleitungen, wie beschrieben, miteinander einen spitzen Winkel einschließen, so dass die Gaszuführungsleitungen an ihren distalen Enden in distaler Richtung divergieren. Die Sitze sind vorzugsweise derart ausgebildet, dass diese einen bestimmten Betrag des Winkels zwischen den Enden der Gaszuführungsleitungen vorgeben. Zudem sind die Sitze dazu eingerichtet, dass die Gaszuführungsleitungen in den Sitzen derart angeordnet werden können, dass zwischen dem distalen Ende der ersten Gaszuführungsleitung und dem distalen Ende der zweiten Gaszuführungsleitung der Freiraum angeordnet ist, der eine Sicht zwischen den Enden der Gaszuführungsleitungen hindurch auf das Gewebe vor den distalen Enden der Gaszuführungsleitungen gestattet.

Die Sitze des Verbindungselements können Mittel zum axialen Fixieren der Gaszuführungsleitungen in dem Verbindungselement aufweisen. Die Sitze können dazu bspw. Formschlussverbindungsmittel, wie etwa einen oder mehrere Anschläge oder Mittel zur Ausbildung einer Schnapp-RastVerbindung und/oder Mittel zur Ausbildung einer Reibschlussverbindung zwischen der Gaszuführungsleitung und dem Verbindungselement aufweisen. Vorzugsweise sind die Sitze dazu eingerichtet, einen bestimmten Betrag des Abstands der Enden der Gaszuführungsleitungen zueinander und/oder in Bezug auf das distale Ende des Verbindungselements vorzugeben. Vorzugsweise sind die Verbindungsmittel dazu eingerichtet, dass die Verbindung zwischen einer Gaszuführungsleitung und dem Verbindungselement hergestellt und/oder gelöst werden kann, während keine Verbindung und/oder eine Verbindung zwischen der anderen Gaszuführungsleitung und dem Verbindungselement hergestellt ist.

Der Freiraum zwischen den distalen Enden der Gaszuführungsleitungen ist vorzugsweise quer zur Richtung distal beidseitig offen, um von außerhalb des Instruments zwischen den Enden der Gaszuführungsleitungen hindurch auf den Bereich des Gewebes vor dem Instrument blicken zu können. Alternativ oder zusätzlich erstreckt sich der Freiraum vorzugsweise von dem distalen Ende der ersten Gaszuführungsleitung bis zum dem distalen Ende der zweiten Gaszuführungsleitung. Alternativ oder zusätzlich verbreitert sich der Freiraum vorzugsweise zwischen den Gaszuführungsleitungen in Richtung distal. Der Freiraum kann sich in Richtung distal beispielsweise in dem spitzen Winkel verbreitern, den das distale Ende der ersten Gaszuführungsleitung mit dem distalen Ende der zweiten Gaszuführungsleitung einschließt.

In einem System ist das erfindungsgemäße Instrument außen an einem Endoskop befestigt. Das Endoskop kann bspw. einen Arbeitskanal aufweisen, wobei das Instrument vorzugsweise außerhalb des Arbeitskanals angeordnet ist. Das Instrument kann außen an dem Endoskop geführt sein. Dadurch kann das Instrument beispielsweise entlang der Achse des distalen Endes des Endoskops verschoben werden. An dem distalen Ende des Endoskops kann eine Lichtquelle zum Beleuchten des Gewebes vor dem Instrument und/oder ein Eingang für eine ein Bild zu dem proximalen Ende des Endoskops übertragende Einrichtung (Bildleiter) aufweisen. Das Instrument kann an dem Endoskop derart angeordnet sein, dass der Benutzer mit Hilfe des Bildleiters zwischen den Enden der Gaszuführungsleitungen hindurch auf das Gewebe vor dem Instrument blicken kann.

Eine Verschiebbarkeit des distalen Endes des Instruments zur Ablation in Axialrichtung, d.h. in Längsrichtung des Endoskops, erleichtert die Handhabung insbesondere bei der Ablation von Gewebeschichten in großen Hohlorganen, wie beispielsweise Ablation der Mucosa im Magen. Das distale Ende des Instruments kann zum Beispiel zwischen 0 mm und 100 mm ausgefahren werden, wobei eine Ausfahrbarkeit bis 50 mm als vorteilhaft, weiter werden 30mm Ausfahrlänge als optimal angesehen.

Es wird als besonders vorteilhaft angesehen, wenn eine dem Verbindungselement zugeordnete, an dem Endoskop befestigte Fassung eine zu einem unrunden Querschnitt des Verbindungselements komplementäre Ausnehmung aufweist, in die das Verbindungselement bei Rückzug in proximaler Richtung einfahren kann. Der unrunde Querschnitt kann insbesondere an dem distalen Ende des Verbindungselements ausgebildet sein. Das Verbindungselement bzw. das Instrumentenende wird dadurch in der Fassung in seiner Drehbeweglichkeit eingeschränkt. Dies erleichtert die Handhabung des erfindungsgemäßen Ablationsinstruments erheblich. Insbesondere kann sichergestellt werden, dass das Verbindungselement bzw. das Instrumentenende durch Rückzug in einer bestimmten Drehlage angeordnet ist, die es auch nach dem Ausschieben beibehalten kann. Das Verbindungselement bzw. das Instrumentenende kann vorzugsweise auch in dem ausgefahrenen Zustand z.B. durch eine Erhebung am Schlauch am proximalen Ende der Fassung und eine dafür vorgesehene Nut in eine gewünschte Lage gebracht werden.

Das erfindungsgemäße Instrument, das Verbindungselement, das System sowie das Verfahren können mit einem oder mehreren der hierin beschriebenen Merkmale weiter gebildet werden.

Die erste Gaszuführungsleitung und die erste Elektrode können zu einer ersten Sonde und die zweite Gaszuführungsleitung und die zweite Elektrode können zu einer anderen zweiten Sonde gehören, wobei die Sonden dazu eingerichtet sein können, dass diese getrennt, unabhängig von der jeweils anderen Sonde zur Plasmabehandlung verwendet werden können. Die erste Sonde und die zweite Sonde können mit dem Verbindungselement derart gehalten werden, dass das distale Ende der Gaszuführungsleitung der ersten Sonde mit dem distalen Ende der Gaszuführungsleitung der zweiten Sonde den spitzen Winkel einschließt, und sodass zwischen dem distalen Ende der ersten Gaszuführungsleitung und dem distalen Ende der zweiten Gaszuführungsleitung der Freiraum angeordnet ist.

Wenigstens eine der Sonden ist mit dem Verbindungselement in dem Instrument vorzugsweise auswechselbar befestigt oder in der Anordnung befestigbar. Auf diese Weise kann der Benutzer eine oder mehrere der mittels des Verbindungselements zu einem Instrument angeordneten Sonden gegen eine von dem Benutzer gewünschte Sonde austauschen.

In dem erfindungsgemäßen Instrument können die Enden der Gaszuführungsleitungen distal über das Verbindungselement überstehen. Das Verbindungselement kann dadurch bei einer Behandlung mit Hilfe des Instruments außerhalb eines Bereichs erhöhter Temperatur, bspw. außerhalb eines Bereichs mit einer Temperatur angeordnet sein, die die Temperatur übersteigt bis zu der das Material, aus dem das Verbindungselement besteht, beständig ist. Die Sitze des Verbindungselements für die Gaszuführungsleitungen können derart ausgebildet sein, dass diese die Position einer Gaszuführungsleitung in dem Instrument bezüglich der anderen Gaszuführungsleitung festlegen. Die Sitze können entsprechend einen distalen Überstand der Enden der Gaszuführungsleitungen über das distale Ende des Verbindungselements festlegen. Die Elektroden sind vorzugsweise außerhalb des Verbindungselements angeordnet. Dies kann die thermische Belastung des Verbindungselements vermindern. Durch diese Maßnahme und/oder eine proximal vor dem Ende der Gaszuführungsleitungen zurückgesetzte Anordnung des Verbindungselements kann das Verbindungselement aus Material hergestellt sein, das eine relativ geringe thermische Belastbarkeit aufweist. Mit der Anordnung der Elektroden außerhalb des Verbindungselements kann auch eine Vermeidung von Kriechströmen zwischen den Elektroden über das Verbindungselement getroffen sein. Die Gefahr von Spannungsüberschlägen ist damit vermindert.

Als Material für das Verbindungselement kommt beispielsweise Keramik oder Kunststoff, wie beispielsweise PEEK, PA, oder Elastomer, wie etwa Silikon, in Frage.

Die erste Gaszuführungsleitung weist vorzugsweise eine Kunststoffleitung auf, die sich von proximal vor dem Verbindungselement durch das Verbindungselement bis distal hinter das Verbindungselement unterbrechungsfrei erstreckt. Genauso weist die zweite Gaszuführungsleitung vorzugsweise eine Kunststoffleitung auf, die sich von proximal vor dem Verbindungselement durch das Verbindungselement bis distal hinter das Verbindungselement unterbrechungsfrei erstreckt. Wenn in den Gaszuführungsleitungen elektrischen Leitungen für die HF-Versorgung der Elektroden geführt sind, so sind diese in den Kunststoffleitungen von dem Verbindungselement und/oder gegeneinander vorzugsweise elektrisch isoliert. Zudem ist die Gefahr von Leckagen in den Gaszuführungsleitungen innerhalb des Verbindungselements mit unerwünschtem Austritt von Gas vermindert, wenn die Gaszuführungsleitungen gesondert von dem Verbindungselement sind, also die Gaszuführungsleitungen nicht abschnittsweise von dem Verbindungselement gebildet sind.

Die Gaszuführungsleitungen sind zumindest an ihren distalen Enden vorzugsweise aus einem hitzebeständigen Kunststoff oder Keramik ausgebildet. Dies ermöglicht Dauerbetrieb der Ablationsvorrichtung, insbesondere zum Abtragen größerer Bereiche der Mucosa.

Vorzugsweise weist die erste Gaszuführungsleitung an ihrem distalen Ende ein Keramikrohr auf und die zweite Gaszuführungsleitung weist an ihrem distalen Ende vorzugsweise ein Keramikrohr auf. Die distalen Enden der Gaszuführungsleitungen können damit besonders hitzebeständig ausgebildet sein. Zudem sind mit den Keramikrohren gebildeten Enden der Gaszuführungsleitungen besonders steif.

Die erste Gaszuführungsleitung und/oder die zweite Gaszuführungsleitung kann an ihrem distalen Ende beispielsweise ein Keramirohr aufweisen, das in einer Kunststoffleitung, beispielsweise einem Kunststoffrohr oder einem Kunststoffschlauch, der Gaszuführungsleitung steckt. Die Keramikrohre können mit ihren distalen Enden aus den Kunststoffleitungen distal herausschauen, so dass die Gaszuführungsleitungen an ihren distalen Enden jeweils durch das Keramikrohr gebildet sind. Auf Grund der entsprechend gegenüber dem distalen Ende der Keramikrohre in proximaler Richtung zurückgesetzt angeordneten distale Enden der Kunststoffleitungen sind die Enden der Gaszuführungsleitungen besonders hitzebeständig. Die distalen Enden der Keramikrohre stehen an den Enden der Gaszuführungsleitungen vorzugsweise distal über die distalen Enden der Kunststoffleitungen um einen Betrag gerade so über, dass die thermische Belastung der distalen Enden der Kunststoffleitungen beim elektrochirurgischen Betrieb des Instruments ein vorgegebenes Maß, beispielsweise eine bestimmte Temperatur, nicht überschreitet. Das distale Ende des Kunststoffschlauchs bzw. des Kunststoffrohrs ist vorzugsweise außerhalb einer Temperaturzone an dem distalen Ende der Gaszuführungsleitung am Rand der Temperaturzone angeordnet, wobei in der Temperaturzone beim elektrochirurgischen Betrieb des Instruments eine Temperatur herrscht, die die Temperatur bis zu der die Kunststoffleitung beständig ist, übersteigt.

Die Kunststoffleitungen bestehen vorzugsweise aus einem Material mit antihaftender, vorzugsweise glatter, Oberfläche, z.B. PTFE, um Gewebeanhaftungen zu vermeiden. Über die distalen Enden der Kunststoffleitungen überstehende Bereiche der Keramikrohre können eine nachbearbeitete Oberfläche aufweisen oder mit einer Antihaftbeschichtung versehen sein, um Gewebeanhaftungen zu vermeiden.

Die erste und zweite Elektrode sind vorzugsweise in den Keramikrohren angeordnet und darin befestigt. Die Enden der Elektroden können distal aus den Keramikrohren herausschauen oder innerhalb der Keramikrohre angeordnet sein.

Vorzugsweise sind die Keramikrohre mit ihren proxima-len Enden in dem Verbindungselement angeordnet. Dies trägt zur Stabilität der Anordnung der Enden der Gaszuführungsleitungen bei. Die Keramikrohre können beispielsweise in dem Verbindungselement stecken, um die Wände der Kunststoffleitungen zumindest an dem distalen Ende des Verbindungselements jeweils zwischen Keramikrohr und Verbindungselement einzuklemmen, um die Gaszuführungsleitungen axial in dem Verbindungselement zu fixieren.

Das Instrument kann eine weitere Sonde, beispielsweise eine Fluidstrahlsonde, insbesondere eine Flüssigkeitsstrahlsonde, aufweisen. Die weitere Sonde kann relativ zu der Anordnung der ersten Gaszuführungsleitung und der zweiten Gaszuführungsleitung bis auf höchstens einen Freiheitsgrad relativ zu der Anordnung fixiert angeordnet sein. Die weitere Sonde kann in einem Sitz des Verbindungselements für die weitere Sonde angeordnet sein. Bevorzugt ist, die weitere Sonde relativ zu der Anordnung der ersten Gaszuführungsleitung und der zweiten Gaszuführungsleitung in und/oder aus dem Freiraum in distaler Richtung verschiebbar. Das weitere Instrument kann beispielsweise in einem Sitz des Verbindungselements verschiebbar geführt sein. Besonders bevorzugt ist die weitere Sonde über das distale Ende der Plasmabehandlungseinheit des Instruments hinaus distal verschiebbar. Das distale Ende der weiteren Sonde kann somit auf das Gewebe aufgesetzt werden, ohne dass die distalen Ende der Gaszuführungskanäle und/oder die Elektroden auf das Gewebe aufgesetzt werden müssen. Das distale Ende des weiteren Instruments kann vorzugsweise in proximaler Richtung hinter die distalen Enden der Gaszuführungskanäle zurückgezogen werden, um eine Sicht auf noch weitere Bereiche des Gewebes zwischen den Enden der Gaszuführungsleitungen hindurch zu gestatten. Zudem ist das distale Ende der weiteren Sonde in einer solchen Ruhestellung thermisch weniger belastet.

Zur Erleichterung der Handhabung des Instruments kann das Instrument ein Gleitelement zum Führen des Instruments über das Gewebe aufweisen. Das Gleitelement kann beispielsweise an dem Verbindungselement befestigt sein. Das Gleitelement ist derart an dem erfindungsgemäßen Instrument angeordnet, so dass das Gleitelement bei der Anwendung des erfindungsgemäßen Instruments zwischen dem Gewebe und dem Instrument angeordnet ist. Das Gleitelement ist derart angeordnet, dass das erfindungsgemäße Instrument bei der Anwendung des Instruments über das Gleitelement auf dem Gewebe, beispielsweise proximal vor dem Einwirkungsbereich des Instruments, aufliegen kann. Die distalen Enden der Plasmasonden können damit in einem festgelegten Abstand von dem Gewebe, beispielsweise mit einem Abstand zwischen 0-10mm, bevorzugt von 0-5mm, besonders bevorzugt 0-3mm, über das Gewebe geführt werden. Der Winkel zwischen der Längsachse des distalen Instrumentenendes und dem Gewebe beträgt dabei bevorzugt zwischen 0° und 80°, besonders bevorzugt zwischen 20° und 30°.

Das erfindungsgemäße Instrument ist vorzugsweise ein monopolares Instrument. Dies bedeutet, dass in diesem Fall die Neutralelektrode nicht an dem Instrument vorgesehen ist, sondern großflächig an dem Patienten befestigt wird. Der Strom fließt dabei nicht über das Instrument sondern über den Körper des Patienten in die Neutralelektrode ab.

Die beiden Elektroden an dem distalen Ende des Instruments sind vorzugsweise elektrisch gegeneinander isoliert und an gesonderte Zuleitungen zur Versorgung der Elektroden mit HF-Leistung angeschlossen. Die Zuleitungen sind bevorzugt durch die Gaszuführungsleitungen geführt.

Bei der Verwendung des erfindungsgemäßen Instruments werden die beiden Elektroden bevorzugt gepulst mit HF-Leistung beaufschlagt. Besonders bevorzugt werden die Elektroden bei Verwendung des erfindungsgemäßen Instruments abwechselnd mit elektrischer Leistung beaufschlagt, wodurch sich ein besonders gleichmäßiger Ablationseffekt und eine über die Breite des erzeugten Ablationsstreifens gemessen einheitliche Koagulationstiefe erreichen lässt. Dies insbesondere, wenn an den beiden Elektroden alternierend hochfrequente Spannung im Bereich mehrerer Hundert Kilohertz anliegt, wobei die Umschaltfrequenz (Alternierungsfrequenz) zwischen beiden Elektroden wenige Hertz beträgt. Die an die Elektroden abgegebene Leistung kann beispielsweise zwischen 10 W und 400W, insbesondere zwischen 80 W und 120 W betragen.

Bei der Verwendung des erfindungsgemäßen Instruments kann bspw. mit der Fluidstrahlsonde vor der thermischen Ablation der Mucosa Flüssigkeit, beispielsweise NaCl-Lösung, in die Magenwand so eingebracht werden, dass sich unter der gewünschten Ablationsstelle ein Flüssigkeitskissen bildet. Die Mündung der Fluidstrahlsonde kann daraufhin vorzugsweise in proximaler Richtung hinter das distale Ende der Anordnung von Plasmasonden zurückgezogen werden.

Weitere Einzelheiten vorteilhafter Ausführungsformen der Erfindung sind Gegenstand der nachfolgenden Beschreibung sowie der Zeichnungen.

Es zeigen schematisch:
Figur 1 - ein erfindungsgemäßes Instrument an einem Endoskop im Einsatz in schematischer Darstellung,
Figur 2 - ein Ende eines Systems mit einem Endoskop und einem daran angeordneten erfindungsgemäßen Instrument zur Ablation in ausschnittsweiser Seitenansicht,
Figur 3a, 3b, 3c - das Endoskop mit dem Ablationsinstrument nach Figur 2 in perspektivischer Ansicht,
Figur 3d - die Fassung für das Instrument gemäß Figur 3a-c in einer Ansicht auf das proximale Ende der Fassung,
Figur 4 - das Endoskop und das Ablationsinstrument nach Figur 2 und 3 in Stirnansicht,
Figur 5 - ein Beispiel eines erfindungsgemäßen Ablationsinstruments in ausschnittsweiser Schnittdarstellung,
Figur 6 - das erfindungsgemäße Ablationsinstrument nach Figur 5 in ausschnittsweiser Draufsicht,
Figur 7 - die elektrische Beschaltung des Ablationsinstruments als Schaltschema,
Figur 8a - eine Darstellung eines Blicks durch einen Bildleiter eines Endoskops auf ein erfindungsgemäßes Instrument und den Behandlungsbereich,
Figur 8b - eine schematische Darstellung des Aufbaus einer Magenwand,
Figur 9 - eine stark schematische Darstellung eines erfindungsgemäßen Instruments an einem Endoskop,
Figur 10a, b, c - ein Ausführungsbeispiel des erfindungsgemäßen Instruments.

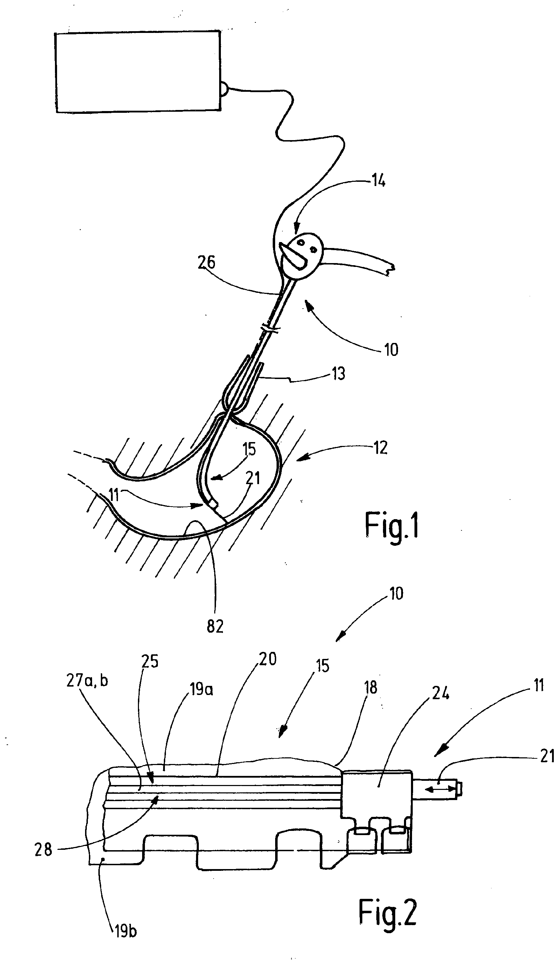

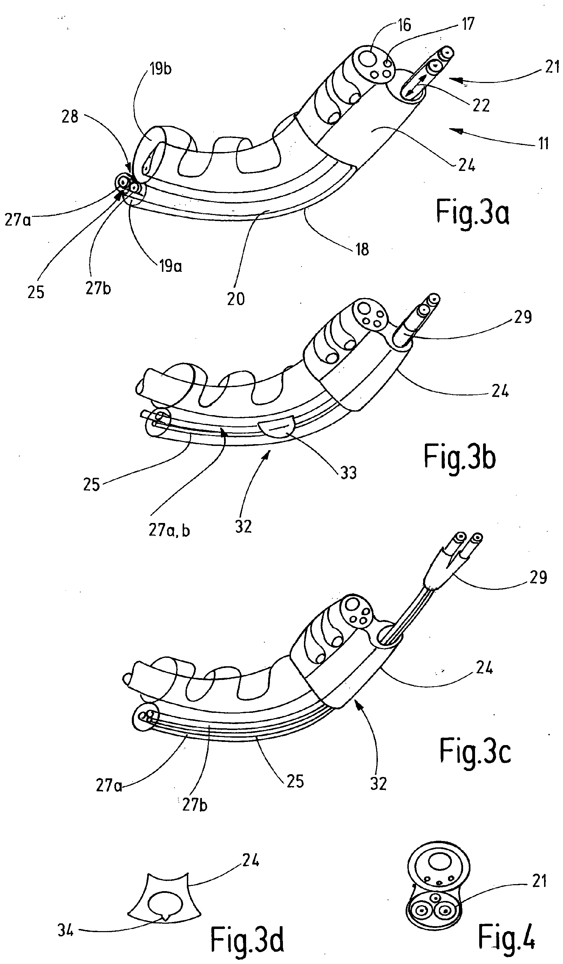

In Figur 1 ist das erfindungsgemäße, an einem Endoskop 10 angebrachte Instrument 11 zur Ablation während des Vorgangs der Mucosaablation an einem Magen 12 veranschaulicht. Zur Behandlung werden das Endoskop 10 und das Instrument 11 durch die Speiseröhre 13 in den Innenraum des Magens 12 eingeführt. Bedienelemente 14 gestatten dabei die Bewegung des Endoskops 10 so, dass sich beispielsweise ein distales Ende 15 des Endoskops gezielt krümmen und/oder entlang der Mageninnenwand bewegen lässt, wodurch verschiedene Stellen der Mageninnenwand leicht erreichbar sind. Das Endoskop 10 kann einen oder mehrere Kanäle 16 (siehe beispielsweise Fig. 3a) enthalten, in die Arbeitsmittel wie bspw. chirurgische Instrumente eingeführt werden können und/oder über die gasförmige und flüssige Fluide zu- oder abgeführt werden können. Das Endoskop 10 kann Mittel zur Bildübertragung 17 enthalten, um die Behandlung optisch kontrollieren zu können.

Die Figuren 2 - 4 veranschaulichen das distale Ende 15 des Endoskops 10 mit dem daran befestigten Instrument 11. Das Instrument 11 kann zusammen mit dem Endoskop 10 in einer Schlauchhülle 18 angeordnet sein, die ein erstes Lumen 19a für das Instrument 11 und ein zweites Lumen 19b für das Endoskop 10 enthält. Das erste und/oder das zweite Lumen 19a,b können, wie in Figur 2 und 3 veranschaulicht, geschlitzt ausgebildet sein, um ein Krümmen des Endoskops 10 zu erleichtern. Die Schlauchhülle 18 kann aus einer dünnen Kunststofffolie bestehen, die das Endoskop 10 und das sich durch einen Ummantelungsschlauch 20 erstreckende Instrument 11 mit entsprechendem Spiel umschließt.

Das Instrument 11 ist im vorliegenden Ausführungsbeispiel bevorzugt als an dem Endoskop 10 außen angebrachtes, sich längs desselben erstreckendes Instrument 11 ausgebildet. Alternativ kann das Instrument 11 beispielsweise auch als innerhalb eines Kanals 16 des Endoskops 10 angeordnetes Instrument 11 ausgebildet sein. Das Instrument 11 weist ein distales Instrumentenende 21 (Instrumentenkopf) auf, das vorzugsweise beweglich gelagert, vorzugsweise in Axialrichtung 22 beweglich ist, die mit der Längsrichtung des distalen Endes 15 des Endoskops 10 übereinstimmt. Das Instrumentenende 21 kann vorzugsweise zusätzlich um die Längsachse 23 des Instrumentenendes, um beispielsweise +/-90°, gedreht werden. Dem Instrumentenende 21 kann eine Fassung 24 zugeordnet sein, die von dem Endoskop 10 gehalten ist. Die Fassung 24 weist eine Durchgangsöffnung auf, in die das Instrumentenende 21 eingefahren werden kann.

Ein in dem zweiten Lumen 19b angeordneter Ummantelungsschlauch 20 ist axial relativ zu dem Endoskop 10, beispielsweise an der Fassung 24, fixiert. Der Ummantelungsschlauch 20 ist an seinem distalen Ende vorzugsweise spiralförmig eingeschnitten, z.B. über eine Länge von etwa 200 mm. In dem als Führungsschlauch dienenden Ummantelungsschlauch 20 sind vorzugsweise wenigstens zwei Gaszuführungsleitungsschläuche 27a,b zur Gasversorgung des Instruments 11 angeordnet. Zudem ist in dem Ummantelungsschlauch 20 vorzugsweise ein längliches Bewegungsübertragungselement 25, z.B. eine Metallwendel, angeordnet, das zum Verschieben und Drehen des Instrumentenkopfes 21 dient. Das Instrumentenende 21 kann beispielsweise in Axialrichtung 22 hin und her bewegt werden, indem das mit dem Instrumentenkopf 21 verbundene Bewegungsübertragungselement 25 hin- und her bewegt wird. In dem Ummantelungsschlauch 20 kann zusätzlich eine Flüssigkeitszuführungsleitung 28 zu dem Instrumentenkopf 21 geführt sein.

Das Instrumentenende 21 ist in Figur 4 in Draufsicht veranschaulicht. Das Instrumentenende 21 weist ein Verbindungselement 29 auf, dessen Aufgabe weiter unten erläutert ist und das einen von der Kreisform abweichenden Querschnitt aufweisen kann. Jedenfalls sind die Abweichungen des Querschnitts der Durchgangsöffnung der Fassung 24 und die Form des Verbindungselements 29 derart, dass das Verbindungselement 29 beim Rückziehen in die Durchgangsöffnung der Fassung 24 findet und sich dabei in die gewünschte Winkelposition dreht.

Um dies zu bewirken, kann das Verbindungselement 29 an seinem proximalen Ende 30 einen Kreisquerschnitt aufweisen. Es kann dort beispielsweise ein Kreiskegel ausgebildet sein. Der Querschnitt des Kegels geht dann ausgehend von dem proximalen Ende 30 in Richtung des distalen Endes 31 allmählich in die aus Figur 4 ersichtliche, von der Kreisform abweichende Form über, wobei die Außenfläche des Verbindungselements 29 vorzugsweise frei von Stufen ist, die an der Mündung der Durchgangsöffnung der Fassung 24 aufsetzen und somit das Rückziehen des Verbindungselements 29 behindern könnten.

Diese Bauform trägt erheblich zur Handhabungserleichterung des Instruments 11 bei. Wird das Instrumentenende 21 bzw. das Verbindungselement 29 so weit aus der dem Verbindungselement 29 zugeordneten Fassung 24 herausgeschoben, so dass lediglich noch das proximale Ende 30 des Verbindungselements 29 in der Fassung 24 steht oder dass das Verbindungselement 29 ganz aus der Fassung 24 herausgeschoben ist, kann das Ende 21 des Instruments 11 über ein Drehen des Bewegungsübertragungselements 25 in verschiedene Winkelpositionen gedreht werden. Wird das Ende 21 des Instruments 11 jedoch zurückgezogen, findet das Verbindungselement 29 in die Fassung 24 ein, die das Verbindungselement 29 und damit das Instrumentenende 21 in die vorgegebene Winkelposition dreht. Die Überführung des Instrumentenendes 21 bzw. des Verbindungselements 29 in die in Figur 3 und 4 veranschaulichte Soll-Position ist somit vorzugsweise automatisch durch Formschluss zwischen dem Verbindungselement 29 und der Fassung 24 gegeben. Die Form des Verbindungselements 29 und der Fassung 24 sind so aufeinander abgestimmt, dass das Verbindungselement 29 nicht nur in seiner Endposition, wenn es ganz in die Fassung 24 eingezogen ist, in radialer Richtung fixiert ist, sondern diese Orientierung im Wesentlichen beibehält, bis das Verbindungselement 29 komplett aus der Fassung 24 austritt. Dadurch ist es möglich, das Instrumentenende 21 in axialer Richtung zu verschieben, und trotzdem behält es seine Rotationsorientierung im Wesentlichen bei. Die Länge der axialen Verschiebung wobei das Verbindungselement 29 und damit das Ende 21 des Instruments 11 seine Position in radialer Richtung im Wesentlichen beibehält, beträgt beispielsweise 15 mm, vorzugsweise 10 mm, besonders bevorzugt 8 mm.

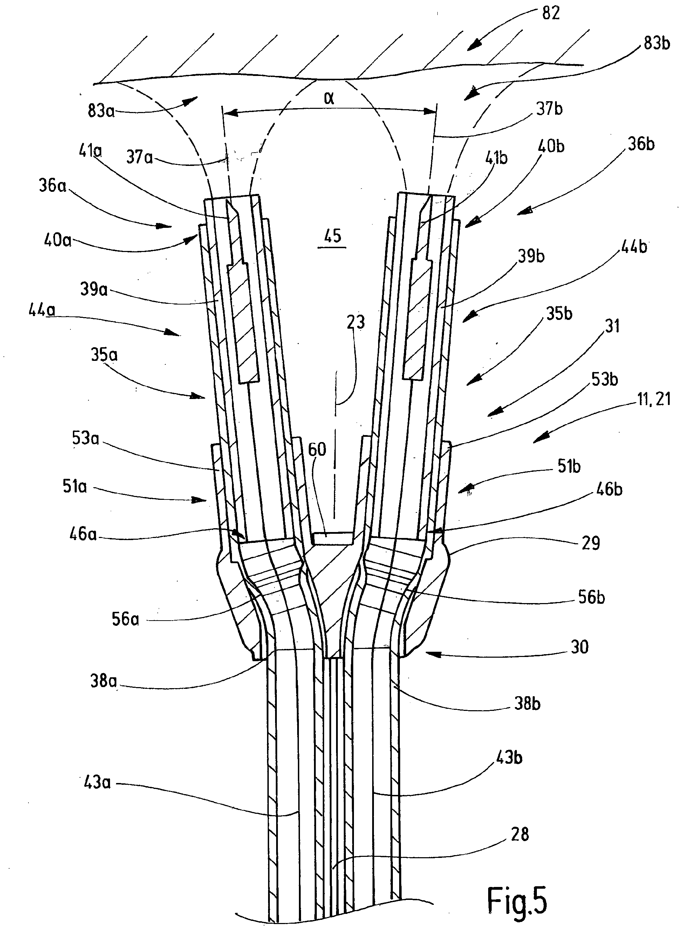

Das Verbindungselement 29 kann bspw. aus Keramik, vorzugsweise aber aus Kunststoff bereitgestellt sein. Wie im Zusammenhang mit Figur 5 beschrieben ist, kann das Verbindungselement 29 insbesondere aus einem Material hergestellt sein, das bei den im Plasmabereich herrschenden Temperaturen nicht beständig und/oder nicht formstabil ist.

Um die Orientierung des Instrumentenendes 21 in radialer Richtung sicherzustellen, wenn das Verbindungselement 29 in axialer Richtung soweit bewegt worden ist, dass es aus der Fassung 24 austritt, kann die Anordnung aus Bewegungsübertragungselement 25 und Gas zuführungsleitungsschläuchen 27a,b mit einer Verdrehsicherung 32 gemäß Figur 3b und 3c ausgebildet sein. In den Figuren 3b und 3c sind der Ummantelungsschlauch 25 sowie die Flüssigkeitszuführungsleitung 28 der Übersichtlichkeit halber nicht dargestellt. Die Verdrehsicherung 32 weist dazu eine vorstehende Formnase in Form einer Feder 33 auf. Figur 3d zeigt die Fassung 24 mit einer in der Fassung 24 vorgesehenen Nut 34 an dem Kanal, durch den sich die Gaszuführungsleitungen 27a,b sowie das Bewegungsübertragungselement 25 und die Flüssigkeitszuführungsleitung 28 erstrecken können. Die Feder 33 taucht in die Nut 34 ein und sichert somit im ausgefahrenen Zustand des Instrumentenendes 21 die gewünschte Lage. Figur 3c zeigt den Instrumentenkopf 21 in einer Position, in der der Instrumentenkopf 21 auf Grund der Feder 33 in der Nut 34 nicht verdreht werden kann.

Die Verdrehsicherung 32 kann bspw. als Kunststoffumspritzung ausgeführt sein. Die Verdrehsicherung 32 kann so ausgebildet sein, dass sie zusammen mit der Fassung 24 einen Endanschlag bildet, der die maximale Länge begrenzt, die das Verbindungselement 29 bzw. das Instrumentenende 21 in axialer Richtung bewegt werden kann. Es ist möglich, die Verdrehsicherung 32 in einem Abstand zur Fassung 24 an dem Ummantelungsschlauch 20 anzuordnen, so dass dadurch die Rotationsorientierung des Verbindungselements 29 und damit des Instrumentenendes 21 in jeder Axialposition sichergestellt ist. Dazu ist es dann erforderlich, dass kurz vor dem Verlassen des Verbindungselements 29 aus der Fassung 24 die Feder 33 bereits in die Nut 34 eingreift oder wenigstens teilweise eingreift.

Es ist auch möglich, die Verdrehsicherung 32 so anzuordnen, dass das Verbindungselement 29 bzw. das Instrumentenende 21 zwischen seinen Endbereichen in Rotationsrichtung frei beweglich gehalten ist. Bei solch einer beispielhaften Anordnung ist eine Rotationsfixierung des Instrumentenendes 21 so lange gewährt, bis das Verbindungselement 29 die Fassung 24 verlässt, und dann wieder, wenn die Feder 33 in die Nut 34 eintritt. Bei einer gesamten Axialbewegung des Verbindungselements 29 bzw. des Instrumentenendes 21 von bspw. 50 mm kann der Bereich mit fixierter Rotationsrichtung des Instrumentenendes 21 in den Bereichen seiner jeweiligen Endlagen jeweils ca. 15 mm einnehmen. Dazwischen kann das Instrumentenende 21 ca. 20 mm auch in radialer Richtung frei beweglich gehalten sein.

Eine längsgeschnittene Darstellung eines Ausführungsbeispiels des erfindungsgemäßen Instruments 11 zeigt Figur 5. Dabei kann es sich beispielsweise um das in den Figuren 1 bis 4 dargestellte Instrument 11 handeln. Das erfindungsgemäße Instrument 11 weist eine erste Gaszuführungsleitung 35a und einen zweite Gaszuführungsleitung 35b auf, die derart relativ zueinander angeordnet sind, dass die distalen Enden 36a,b der Gaszuführungsleitungen 35a,b miteinander einen spitzen Winkel α von vorzugsweise zwischen minimal 1° und maximal 60°, besonders bevorzugt zwischen minimal 1° und maximal 30°, besonders bevorzugt 10°, einschließen, so dass die Enden 36a,b der Gaszuführungsleitungen 35a,b in Richtung distal divergieren. Die Enden 36a,b der Gaszuführungsleitungen 35a,b weisen dazu unterschiedlich orientierte Mittelachsen 37a,b auf, die in Figur 5 durch strichpunktierte Linien markiert sind. Die Mittelachsen 37a,b schließen den oben genannten Winkel α miteinander ein. Die Enden 36a,b der Gaszuführungsleitungen 35a,b können derart angeordnet sein, dass deren Mittelachsen 37a,b in einer gemeinsamen Ebene liegen.

Die Enden 36a,b der Gaszuführungsleitungen 35a,b können einen Kreisquerschnitt oder auch abweichende Querschnitte, wie einen Ovalquerschnitt, Mehreckquerschnitte, oder dergleichen, aufweisen. Die Gaszuführungsleitungen 35a,b werden vorzugsweise jeweils durch eine Kunststoffleitung 38a,b, z.B. ein flexibles Rohr oder einen Schlauch aus Kunststoff, gebildet. Die Kunststoffleitungen 38a,b können Endabschnitte der Gaszuführungsleitungsschläuche 27a,b sein oder an diese angeschlossen sein. Zusätzlich zu den Kunststoffleitungen 38a,b können zu den Gaszuführungsleitungen 35a,b Keramikrohre 39a,b gehören, wobei jeweils ein Keramikrohr 39a,b in den distalen Enden 40a,b der Kunststoffleitungen 38a,b steckt. Die Gaszuführungsleitungen 35a,b dienen der Zuführung eines Gases, insbesondere eines Inertgases, beispielsweise Argon, an das distale Ende 21 des Instruments 11. Es können jedoch auch Aktivgase, Aerosole oder dergleichen zuzuführen sein, wozu die Gaszuführungsleitungen 35a,b gleichermaßen dienen können.

In dem Ende 36a der ersten Gaszuführungsleitung 35a ist wenigstens eine erste Elektrode 41a angeordnet und in dem Ende 36b der zweiten Gaszuführungsleitung 35b ist wenigstens eine zweite Elektrode 41b angeordnet. In dem dargestellten Ausführungsbeispiel sind die Elektroden 41a,b in den distalen Enden der Keramikrohre angeordnet, die im Ausführungsbeispiel die distalen Enden 36a,b der Gaszuführungsleitungen 35a,b bilden. Die Elektroden 41a,b, die beispielsweise aus einem hitzebeständigen Metall, wie Wolfram, ausgebildet sein können, sind vorzugsweise jeweils in dem Keramikrohr 39a,b befestigt. Dazu können die Elektroden 41a,b beispielsweise jeweils federnd bildende Abschnitte (nicht dargestellt) aufweisen, die sich auf Grund der Federkraft gegen die Innenfläche des Keramikrohrs 39a,b stemmen, so dass die Elektroden 41a,b jeweils durch Reibschluss in dem Keramikrohr 39a,b gehalten sind. Die Elektroden 41a,b sind vorzugsweise mittig in den Öffnungen der Gaszuführungsleitungen 35a,b angeordnet und/oder längs zu den Mittelachsen 37a,b orientiert. Die Elektroden 41a,b können beispielsweise stab-, spatel-, messer- oder nadelförmig sein. Die Spitzen der Elektroden 41a,b können innerhalb der Gaszuführungsleitungen 35a,b liegen, wie es Figur 5 andeutet, oder aus diesen herausschauen. Die erste Elektrode 41a und die zweite Elektrode 41b können miteinander einen spitzen Winkel, vorzugsweise zwischen minimal 1° und maximal 60°, besonders bevorzugt zwischen minimal 1° und maximal 30°, beispielsweise 10°, derart einschließen, dass die erste Elektrode und die zweite Elektrode distal divergieren. Der Betrag des Winkels zwischen den Elektroden 41a,b kann dem Betrag des Winkels a zwischen den Enden 36a,b der Gaszuführungsleitungen 35a,b entsprechen. Der Abstand zwischen den distalen Spitzen der Elektroden 41a,b beträgt vorzugsweise mehrere Millimeter (z.B. 3 mm bis 12 mm), wobei ein Abstand von 5 mm bis 10 mm, insbesondere 6,5 mm besonders vorteilhaft ist, um eine gleichmäßige, breitstreifige Gewebeablation mit homogener Ablationstiefe zu erreichen. Der Durchmesser der Spitzen der Elektroden 41a,b liegt vorzugsweise im Bereich von 0,2 mm bis 1 mm, wobei im vorliegenden Ausführungsbeispiel ein Durchmesser von 0,4 mm gewählt worden ist. Dies hat sich wegen der aufgrund des geringen Durchmessers auftretenden hohen Feldstärken und deswegen der guten Zündwilligkeit der Elektroden 41a,b einerseits und der damit zu erzielenden Gewebeeffekte anderseits als vorteilhaft herausgestellt.

Die Elektroden 41a,b sind vorzugsweise gegeneinander isoliert. Durch die Gaszuführungsleitungen 35a,b erstrecken sich elektrische Leitungen 43a,b zur Versorgung der Elektroden 41a,b mit HF-Leistung. Die Kunststoffleitungen 38a,b isolieren die elektrischen Leitungen 43a,b jeweils gegen die andere elektrische Leitung 43a,b. Die Gaszuführungsleitungen 35a,b mit den Elektroden 41a,b bilden Plasmasonden 44a,b zur elektrochirurgischen Behandlung des Gewebes vor dem Instrument 11.

Die Keramikrohre 39a,b stehen vorzugsweise distal über die Enden 40a,b der Kunststoffleitungen 38a,b hervor. Auf diese Weise sind die Enden 36a,b der Gaszuführungsleitungen 35a,b aus Keramik gebildet und damit besonders temperaturstabil, auch bei Dauerbetrieb des Instruments 11 zur großflächigen Ablation. Außerdem kann damit eine Kriechstrecke zwischen den Elektroden 41a,b verlängert sein, was die Spannungsfestigkeit der Anordnung verbessert. Mit den Keramikrohren kann außerdem die Durchschlagfestigkeit zwischen den Elektroden 41a,b erhöht sein.

Alternativ zu der in Figur 5 dargestellten Anordnung kann die Kunststoffleitung 38a,b und, wenn vorhanden, auch das Keramikrohr 39a,b an dem distalen Ende 36a,b der Gaszuführungsleitung abschließen bzw. gemeinsam abschließen. Insbesondere wenn keine Keramikrohre 39a,b in den Enden 40a,b der Kunststoffleitungen 38a,b stecken, bestehen die Kunststoffleitungen 38a,b oder zumindest deren distale Enden 40a,b vorzugsweise aus hitzebeständigem Kunststoff.

Zwischen den in dem Winkel α gespreizt angeordneten distalen Enden 36a,b der Gaszuführungsleitungen 35a,b ist ein Freiraum 45 angeordnet, um einen gespreizt gabelförmigen Instrumentenkopf 21 zu bilden. Der sich in Richtung distal verbreiternde Freiraum 45 erstreckt sich quer zur Richtung distal bzw. proximal von dem distalen Ende 36a der ersten Gaszuführungsleitung 35a bis zum dem distalen Ende 36b der zweiten Gaszuführungsleitung 35b. Der Freiraum 45 erstreckt sich zwischen den Gaszuführungsleitungen 35a,b von den distalen Enden 36a,b der Gaszuführungsleitungen 35a,b proximal bis zwischen die proximalen Enden 46a,b der Keramikrohre 39a,b. In dem in Figur 5 dargestellten Ausführungsbeispiel erstreckt sich der Freiraum 45 zudem zwischen den Gaszuführungsleitungen 35a,b von dem freien distalen Endabschnitt des Keramikrohrs 39a der ersten Gaszuführungsleitung 35a bis zu dem freien distalen Endabschnitt des Keramikrohrs 39b der zweiten Gaszuführungsleitung 35b. Außerdem erstreckt sich der Freiraum 45 zwischen den Gaszuführungsleitungen 35a,b von dem aus dem Verbindungselement 29 distal hervorragenden Abschnitt der Kunststoffleitung 38a der ersten Gaszuführungsleitung 35a bis zu dem aus dem Verbindungselement 29 distal hervorragenden Abschnitt der Kunststoffleitung 38b der zweiten Gaszuführungsleitung 35b.

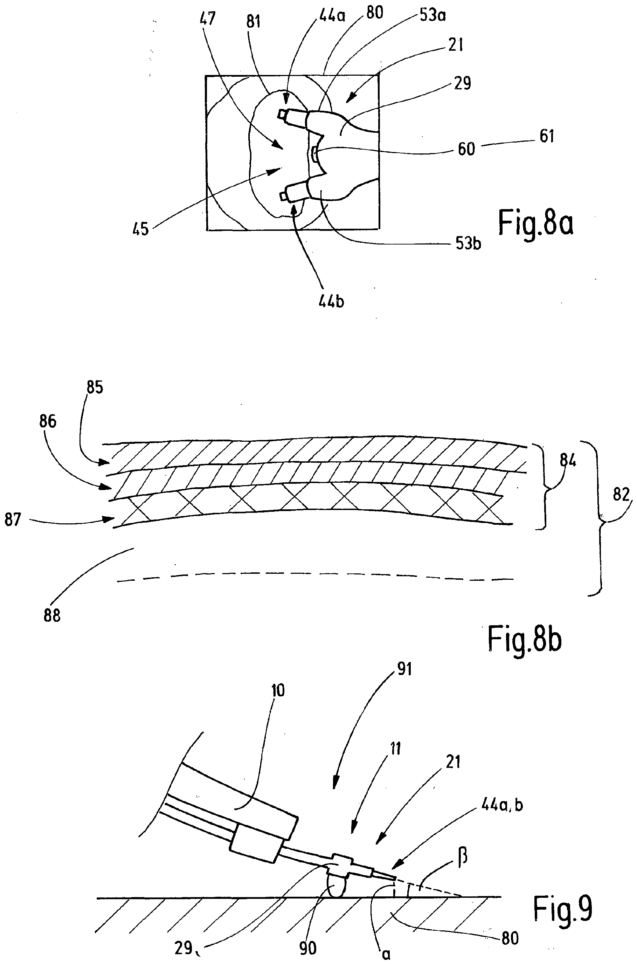

Durch den Freiraum 45 bietet das Instrument 11 dem Benutzer des Instruments 11 eine Sicht zwischen den Enden 36a,b der Gaszuführungsleitungen 35a,b hindurch auf den Ablationsbereich der Plasmasonden 44a,b, insbesondere während der Plasmabehandlung des Gewebes mit dem Instrument 11. Insbesondere kann der Benutzer von außerhalb des Instruments 11, beispielsweise mit Hilfe eines Mittels 17 zur Bildübertragung an oder in dem Endoskop 10, an dem das Instrument 11 angeordnet sein kann, zwischen den Spitzen der Plasmasonden 44a,b durch den Freiraum 45 hindurch auf den Gewebebereich 47 (s. auch Figur 8a) hinter dem Instrument 11 blicken und/oder ein Bild des Gewebebereichs 47 hinter dem Instrument 11 aufnehmen. Da der Benutzer den Einwirkungsbereich des Instruments 11 auf Grund des Freiraums 45 besser einsehen kann, kann dieser das Instrument 11 besonders zügig und trotzdem sicher führen. Zudem weist der Instrumentenkopf 21 auf Grund des Freiraums 45 eine relativ geringe Wärmekapazität an dem distalen Ende auf, das bei Benutzung der Plasmasonden 44a,b heiß werden kann. Dadurch kann das Instrument 11 nach der Benutzung rasch aus dem Körper des Patienten entfernt werden. Unerwünschte Gewebeanhaftungen an dem Ende des Instruments können durch den Freiraum 45 reduziert sein.

Das in Figur 5 dargestellte Instrument 11 weist zur Anordnung der distalen Enden 36a,b der Gaszuführungsleitungen 35a,b zueinander derart, dass diese den Winkel α einschließen, ein Verbindungselement 29 mit Sitzen 51a,b für die Gaszuführungsleitungen 35a,b auf, die dazu eingerichtet sind, die Enden 36a,b der Gaszuführungsleitungen 35a,b in dem Winkel α zueinander zu fixieren. Die Sitze 51a,b geben den Winkel α zwischen den Enden 36a,b der Gaszuführungsleitungen 35a,b vorzugsweise vor, um die Anordnung der Enden 36a,b der Gaszuführungsleitungen 35a,b relativ zueinander beim Zusammenbau des Instruments 11 zu erleichtern.

Um die Enden 36a,b der Gaszuführungsleitungen 35a,b so zu fixieren, dass diese einen festen Abstand voneinander aufweisen, weisen die Sitze 51a,b des Verbindungselements 50 Befestigungsmittel zur axialen Fixierung der Enden 36a,b der Gaszuführungsleitungen 35a,b auf. Die Befestigungsmittel können Mittel zur Ausbildung einer Formschlussverbindung und/oder Mittel zur Ausbildung einer Reibschlussverbindung zwischen den Gaszuführungsleitungen 35a,b und dem Verbindungselement 29 aufweisen. Die Gaszuführungsleitungen 35a,b können in dem Verbindungselement 29 festgeklemmt sein. Das Verbindungselement 29 kann alternativ oder zusätzlich beispielsweise Schnapp-Rast-Verbindungsmittel aufweisen, die mit entsprechenden Mitteln an den Gaszuführungsleitungen 35a,b zusammenwirken. In dem in Figur 5 dargestellten Ausführungsbeispiel sind die Gaszuführungsleitungen 35a,b in dem Verbindungselement 29 mittels Reibschlusses auf Grund eines Übermaßes der Gaszuführungsleitungen 35a,b axial fixiert.

Die Sitze 51a,b können die Abstände der Enden 36a,b der Gaszuführungsleitungen 35a,b voneinander und/oder von dem Verbindungselement 29 vorgeben, um die Anordnung der Enden 36a,b der Gaszuführungsleitungen 35a,b beim Zusammenbau des Instruments 11 zu erleichtern. Zur Festlegung des Abstands können beispielsweise Anschläge oder sonstige Formschlusselemente an dem Verbindungselement 29 und den Gaszuführungsleitungen 35a,b ausgebildet sein.

Durch das Verbindungselement 29 kann ein modularer Aufbau des Instruments 11 verwirklicht sein. Es können beispielsweise verschiedene Sonden 44a,b, insbesondere solche zur Plasmakoagulation, beispielsweise unterschiedlicher Bauformen zueinander angeordnet werden. Mit dem Verbindungselement 29 können einzelne Sonden 44a,b, die jeweils, gesondert von dem Instrument 11, insbesondere außerhalb des Sitzes 51a,b in dem Verbindungselement 29, verwendbar sind (Einzelsonden), zu dem Instrument 11 angeordnet werden. Die distalen Enden der Einzelsonden 44a,b werden dadurch zueinander in dem spitzen Winkel a, der von den Sitzen 51a,b des Verbindungselements 50 vorzugsweise vorgegeben ist, und vorzugsweise in einem Abstand voneinander fixiert angeordnet. Vorzugsweise sind eine oder beide Plasmasonden 44a,b auswechselbar ohne das Verbindungselement 29 zerstören und/oder ohne die auszuwechselnde Plasmasonde 44a,b zerlegen zu müssen. Die mit dem Verbindungselement verbundenen Plasmasonden 44a,b können vorzugsweise jeweils aus dem Verbindungselement 29 entnommen werden, um eine andere Plasmasonde 44a,b in das Instrument 11 einzuwechseln. Die Funktionsfähigkeit der entnommenen Plasmasonde 44a,b bleibt bei der Entnahme vorzugsweise erhalten.

Das in Figur 5 dargestellte Verbindungselement 29 verjüngt sich vorzugsweise in Richtung proximal. Dadurch ergibt sich ein besonders schlanker Aufbau des Verbindungselements 50 und damit des Instrumentenkopfes 21.

Der Freiraum 45 zwischen den distalen Enden 36a,b der Gaszuführungsleitungen 35a,b erstreckt sich vorzugsweise proximal wenigstens bis zu dem distalen Ende 31 des Verbindungselements 50. Das in Figur 5 dargestellte Verbindungselement 29 weist an seinem distalen Ende 31 zwei in dem Winkel α gespreizt angeordnete Fortsätze 53a,b auf, die beispielsweise rohr- oder schlauchförmigen sein können, wobei sich die Gaszuführungsleitungen 35a,b durch die Fortsätze 53a,b erstrecken. Die Fortsätze 53a,b bilden voneinander gesonderte Kanäle in dem Verbindungselement 29, durch die sich die Gaszuführungsleitungen 35a,b zur Anordnung der Enden 36a,b der Gaszuführungsleitungen 35a,b in dem Winkel α und vorzugsweise zur axialen Fixierung mittels Reibschluss erstrecken. Die Mittelachsen der geraden Fortsätze 53a,b schließen den Winkel α miteinander ein und legen dadurch den Winkel α zwischen den Enden 36a,b der Gaszuführungsleitungen 35a,b fest. Der Freiraum 45 erstreckt sich von zwischen den distalen Enden 36a,b der Gaszuführungsleitungen 35a,b in Richtung proximal zwischen die Fortsätze 53a,b, so dass der Benutzer zwischen den Fortsätzen hindurch durch den sich von dem einen Fortsatz 53a zu dem anderen Fortsatz 53b erstreckenden Freiraum 45 auf das Gewebe schauen kann.

In dem in Figur 5 dargestellten Ausführungsbeispiel stecken die Keramikrohre 39a,b mit ihren proximalen Enden 46a,b in den Fortsätzen 53a,b des Verbindungselements 29. Zur axialen Fixierung der Enden 36a,b der Gaszuführungsleitungen 35a,b mittels des Verbindungselements 29 ist deren Kunststoffleitungswand jeweils zwischen Keramikrohr 39a,b und Verbindungselement 29 eingeklemmt. Der innere und äußere Durchmesser der Kunststoffleitungen 38a,b kann an dem distalen Ende 40a,b der Kunststoffleitung 38a,b gegenüber einem Abschnitt vor dem distalen Ende 40a,b der Kunststoffleitung 38a,b erweitert sein, um das Keramikrohr 39a,b aufzunehmen. Die Erweiterung kann durch das Einpressen des Keramikrohrs 39a,b in das Ende 40a,b der Kunststoffleitung 38a,b ausgebildet sein. Die sich von dem distalen Ende 36a,b der Gaszuführungsleitungen 35a,b bis in das Verbindungselement 29 erstreckenden geraden Keramikrohre 39a,b tragen zu einer beruhigten Gasströmung durch die Enden 36a,b der Gaszuführungsleitungen 35a,b bei. Der Gasströmungsquerschnitt in dem Keramikrohr 39a,b und der Gasströmungsquerschnitt der Kunststoffleitung 38a,b proximal vor der Erweiterung sind vorzugsweise gleich.

Wie in Figur 5 dargestellt können die Kunststoffleitungen 38a,b an dem proximalen Ende des Verbindungselements 29 vor dem Verbindungselement 29 annähernd parallel zueinander verlaufen. Wie in Figur 5 dargestellt sind die Gaszuführungsleitungen 35a,b in dem Verbindungselement 29 quer zur Längserstreckung der Gaszuführungsleitungen 35a,b vorzugsweise voneinander beabstandet angeordnet. Wie in Figur 5 ebenfalls dargestellt sind die Gaszuführungsleitungen 35a,b in dem Verbindungselement 29 vorzugsweise derart angeordnet, dass Abschnitte 56a,b der Gaszuführungsleitungen 35a,b, im Ausführungsbeispiel der Kunststoffleitungen 38a,b, innerhalb des Verbindungselements 29 vor den distalen Enden 36a,b der Gaszuführungsleitungen 35a,b einen Winkel einschließen der größer ist als der Winkel a zwischen den Enden 36a,b der Gaszuführungsleitungen 35a,b, um einen Abstand der Enden 36a,b der Gaszuführungsleitungen 35a,b voneinander und damit eine Breite des Freiraums 45 unabhängig von dem Winkel a zwischen den Enden 36a,b der Gaszuführungsleitungen 35a,b bereitstellen zu können. Die Gaszuführungsleitungen 35a,b divergieren in einem Abschnitt in dem Verbindungselement 29 vor den distalen Enden 36a,b der Gaszuführungsleitungen 35a,b nach distal entsprechend zunächst mehr und dann weniger. Dadurch ergibt sich eine schlanke Anordnung der Gaszuführungsleitungen 35a,b vor den Abschnitten. Zudem kann dadurch Platz für einen Kopf 60 einer weiteren Sonde, z.B. einer Flüssigkeitsstrahlsonde 61, geschaffen sein. Der Kopf 60 der weiteren Sonde und die Flüssigkeitszuführungsleitung 28 sind in Figur 5 nichtgeschnitten dargestellt.

Wie dargestellt erstrecken sich die Kunststoffleitungen 38a,b vorzugsweise unterbrechungsfrei durch das Verbindungselement 29. Die Kunststoffleitungen 38a,b isolieren die elektrischen Leitungen 43a,b jeweils gegen das Verbindungselement 29.

Die Enden 36a,b der Gaszuführungsleitungen 35a,b stehen distal über das distale Ende 31 des Verbindungselements 29, im dargestellten Ausführungsbeispiel über die Fortsätze 53a,b, über. Dadurch ist das proximal gegenüber den distalen Enden der Plasmasonden 44a,b zurückgesetzt angeordnete Verbindungselement 29 von der Zone an den distalen Enden 36a,b der Gaszuführungsleitungen 35a,b mit erhöhter Temperatur beabstandet. Das Verbindungselement 29 kann daher aus einem Material gefertigt sein, das den in der Zone während der Plasmabehandlung herrschenden Temperaturen nicht standhält.

Als Material für das Verbindungselement 29 kommt beispielsweise Kunststoff, wie beispielsweise PEEK, PA oder Elastomer, wie beispielsweise Silikon, in Betracht.

Die Elektroden 41a,b sind wie dargestellt vorzugsweise außerhalb des Verbindungselements 29, von dem distalen Ende 31 des Verbindungselements 29 beabstandet angeordnet. Kriechstrecken zwischen den Elektroden 41a,b über das Verbindungselement 29 sind damit besonders lang.

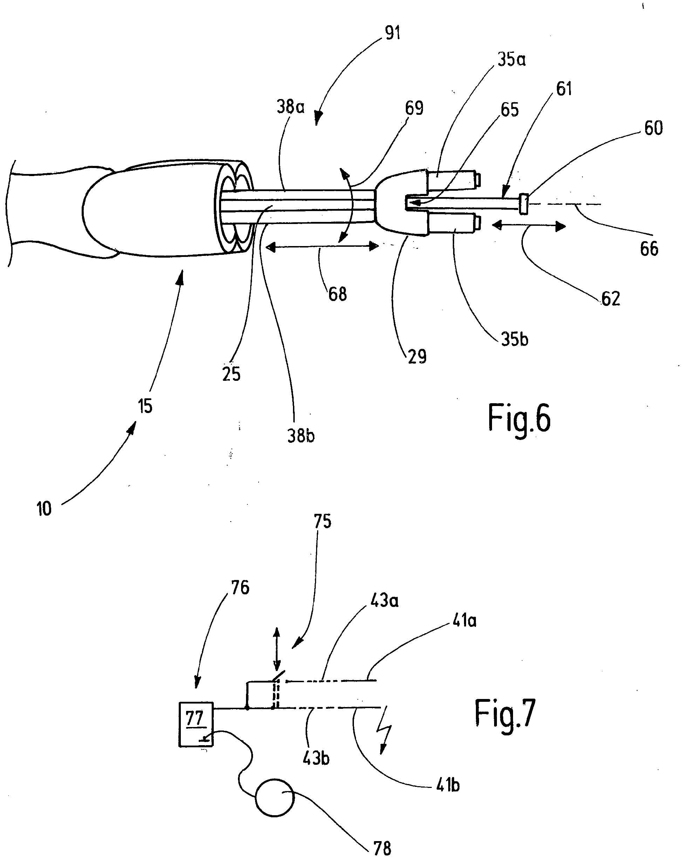

Die erwähnte weitere Sonde 61, die das Instrument 11 zusätzlich zu den Plasmasonden 44a,b aufweisen kann, ist in Figur 5 in einer Ruhestellung und in Figur 6 in einer über die distalen Enden der Plasmasonden 44a,b hinaus vorgeschobenen Arbeitsstellung gezeigt. Die Verschiebbarkeit der weiteren Sonde 61 ist durch den Doppelpfeil 62 angedeutet. Bei der weiteren Sonde 61 kann es sich beispielsweise um eine Flüssigkeitsstrahlsonde zum Applizieren von wässriger NaCl-Lösung unter die Mucosa zum Anheben der Mucosa handeln. Mit dieser kann ein Flüssigkeitsstrahl mit einem zum Beispiel zum Unterspritzen der Mucosa erforderlichen Druck bzw. Flow ausgebildet werden. Die Flüssigkeitsstrahlsonde 61 weist dazu an ihrem Kopf 60 an dem distalen Ende der Flüssigkeitsstrahlsonde eine Düse mit einer Austrittsöffnung auf, durch die eine Flüssigkeit, zum Beispiel Natriumchlorid-Lösung, beispielweise als Strahl ausgestoßen werden kann. Damit lassen sich Gewebepartien bearbeiten, beispielsweise unterspritzen, wenn der Strahl mit entsprechendem Druck, Flow und Form austritt, dass er wie eine Nadel in das Gewebe eindringen kann. Die weitere Sonde ist vorzugsweise in einem von den Sitzen der Gaszuführungsleitungen gesonderten Sitz 65 des Verbindungselements 29 gehalten. Wie erwähnt ist die weitere Sonde 61 in dem Sitz 65 in Richtung distal bzw. proximal relativ zu dem Verbindungselement 29 vorzugsweise verschiebbar geführt. Alternativ dazu kann die weitere Sonde 61 in dem Sitz 65 in Bezug auf das Verbindungselement 29 fixiert sein, so dass eine Bewegung des Kopfes 60 der weiteren Sonde 61 in Bezug auf das Verbindungselement 29 nicht möglich ist.

Die weitere Sonde 61 ist vorzugsweise derart in distale Richtung verschiebbar geführt, dass die Mittelachse 66 der weiteren Sonde 61 oberhalb einer gedachten Linie, die die Mittelpunkte der Öffnungen der Gaszuführungsleitungen 35a,b verbindet, liegt. Die sich in Führungsrichtung erstreckende Mittelachse des Schiebesitzes 65 in dem Verbindungselement 29 für die weitere Sonde 61 ist vorzugsweise oberhalb einer gedachten, quer zur Führungsrichtung orientierten Linie von der Mittelachse des Sitzes 51a für die erste Gaszuführungsleitung 35a zu der Mittelachse des Sitzes 51b für die zweite Gaszuführungsleitung 35b angeordnet. Dies ist auch in dem in Figur 5 und Figur 6 dargestellten Ausführungsbeispiel der Fall, die das Instrument 11 von unten zeigen.

Durch die Verschiebbarkeit der weiteren Sonde 61 entlang der Längsachse 23 des Endes des Instruments relativ zu dem Verbindungselement 29 bzw. relativ zu den distalen Enden der Plasmasonden 44a,b kann der Kopf 60 der weiteren Sonde 61 in Richtung distal relativ zu der Anordnung der Enden 36a,b Gaszuführungsleitungen 35a,b in die erwähnte Arbeitsstellung verschoben werden und der Kopf 60 der weiteren Sonde 61 relativ zu der Anordnung in Richtung proximal in die erwähnte Ruhestellung zurückbewegt werden.

Die weitere Sonde 61 ist vorzugsweise derart angeordnet, dass der Kopf 60 der weiteren Sonde 61 distal durch den zwischen den Enden 36a,b der Gaszuführungsleitungen 35a,b angeordneten Freiraum 45 über die distalen Enden der Plasmasonden 44a,b hinaus in die Arbeitsstellung verschoben werden kann und dass der Kopf 60 der weiteren Sonde 61 durch den Freiraum 45 in eine Ruhestellung proximal vor dem Freiraum 45 zurückbewegt werden kann.

Wie in den Figuren 5 und 6 dargestellt kann an der weiteren Sonde 61 ein Anschlagelement, z.B. wie in den Figuren 5 und 6 von dem relativ zu der Flüssigkeitszuführungsleitung verbreitertem Kopf 60 gebildet, angeordnet sein, das ein zu weites Zurückziehen des Kopfes 60 der weiteren Sonde 61 proximal über die Ruhestellung hinaus verhindert.

Wenn der Kopf 60 der Flüssigkeitsstrahlsonde 61 distal über die Enden der Plasmasonden 44a,b hinaus verschoben ist, kann der Kopf 60 der Flüssigkeitsstrahlsonde 61 auf das Gewebe aufgesetzt werden, um durch die Düse in dem Kopf 60 NaCl-Lösung in das Gewebe zu injizieren, ohne dass dabei gleichzeitig die distalen Enden der Plasmasonden 44a,b das Gewebe berühren. Wird der Kopf 60 der weiteren Sonde 61 in die Ruhestellung proximal vor dem Freiraum 45 zurückgezogen, hat der Benutzer eine noch weiter verbesserte Sicht auf den Ablationsbereich.

An der weiteren Sonde 61 kann ein Reinigungselement (nicht dargestellt) angeordnet sein, mit dem Gewebeanhaftungen oder sonstige Verschmutzungen von den Plasmasonden 44a,b abgeschoben werden können.

Figur 6 zeigt zudem ein Beispiel eines länglichen Bewegungsübertragungselementes 67, das zug-, druck- und torsionssteif, jedoch leicht biegbar ist, und das ein Verschieben (Pfeil 68) des Instrumentenkopfs 21 vor- und zurück in Bezug auf das distale Ende 15 des Endoskops 10 sowie ein Drehen (Pfeil 69) um eine Achse entlang des Instruments 11 erlaubt. Das Bewegungsübertragungselement 67 ist zur Übertragung der Bewegung mit dem Verbindungselement 29 und einem Bedienelement 14 (s. Figur 1) verbunden. Bei dem Bewegungsübertragungselement 67 kann es sich beispielsweise um eine, vorzugsweise elektrisch isolierte, Metallwendel handeln.

Wie in Figur 7 veranschaulicht sind die elektrischen Leitungen 43a,b zur Versorgung der Elektroden 41a,b mit HF-Leistung über eine Schalteranordnung 75 an eine elektrische Quelle 76, zum Beispiel in Gestalt eines Hochfrequenzgenerators 77, angeschlossen. Dieser stellt eine HF-Spannung mit mehreren hundert Kilohertz (zum Beispiel 350 kHz) und einer geeigneten Spannung oberhalb 1000 Vp (z.B. zwischen minimal 4 kVp und maximal 6kVp, vorzugsweise 4,3kVp oder 4,9 kVp). Der HF-Generator 77 kann eine Leistung von mehr als 100 W (beispielsweise 120 W) aufbringen.

Die Spannung wird bezogen auf ein Null-Potential bereitgestellt, an das der Patient über mindestens eine Neutralelektrode 78 angeschlossen ist. Diese Neutralelektrode 78 wird großflächig an einer geeigneten Stelle des Körpers des Patienten angebracht. Die Schalteranordnung 75 verbindet die Leitungen 43a,b und somit die Elektroden 41a,b alternierend, d.h. abwechselnd mit dem Ausgang des Hochfrequenzgenerators 77. Die Schaltfrequenz, mit der die Elektroden 41a,b abwechselnd aktiviert werden, liegt im Bereich einiger Hz, vorzugsweise zwischen 1 Hz und 20 Hz, vorzugsweise 5 Hz.

Die Gaszuführungsleitungen 35a,b können für die Plasmabehandlung mit einem Gasfluss von zusammengenommen zwischen beispielsweise mindestens 1 Liter/Minute bis maximal 4 Liter/Minute, bevorzugt 2 Liter/Minute beaufschlagt werden.

Figur 8a zeigt ein Bild 80, das mit Hilfe des Mittels 17 zur Bildübertragung in dem Endoskop 10 aufgenommen ist. Durch das Endoskop 10 ist der Instrumentenkopf 21 mit dem Verbindungselement 29 und den Enden der Plasmasonden 44a,b und zudem ein durch das Beleuchtungsmittel an dem Endoskop 10 beleuchteter Gewebebereich 47 sichtbar. In dem Gewebebereich 47 ist eine Gewebestelle 81 vorhanden, die mit dem Instrument 11 behandelt werden soll. Der Kopf 60 der Flüssigkeitsstrahlsonde 61 ist proximal aus dem Freiraum 45 in eine Ruhestellung zurückgezogen. Wie erkennbar bietet der gespreizt gabelförmige Instrumentenkopf 21 auf Grund des Freiraums 45 zwischen den Enden der Plasmasonden 44a,b und den Fortsätzen 53a,b durch den Freiraum 45 hindurch eine freie Sicht auf die Gewebestelle 81. Dies erleichtert die sichere und zügige Führung des Instruments 11 erheblich.

Das insoweit beschriebene Instrument 11 zur Ablation arbeitet wie folgt: Zur flächenhaften Ablation der Mucosa, beispielsweise zur therapeutischen Behandlung krankhafter Gewebeveränderungen, zur Beeinflussung des Gewichts- und Essverhaltens von Patienten oder aus anderen therapeutischen Gründen, wird das mit dem Instrument 11 zur Ablation versehene Endoskop 10 gemäß Figur 1 durch die Speiseröhre 13 des Patienten in dessen Magen 12 eingeführt. Mittels der Bedienelemente 14 des Endoskops 10 wird dessen distales Ende 15 an die gewünschte Ablationsstelle so positioniert, dass die zu behandelnde Gewebestelle im Sichtfeld des Endoskops 10 liegt. Es wird nun durch entsprechendes Schieben der Anordnung aus Ummantelungsschlauch und Gaszuführungsleitungsschläuchen 13 und/oder des Bewegungsübertragungselements 67 das distale Ende 21 des Ablationsinstruments 11 etwas vorgeschoben, so dass er im gewünschten Abstand, beispielsweise 3 mm zu der Mucosa 84 steht.

Vor der thermischen Ablation, beispielhaft der Mucosa 84 (s. auch Figur 8b), wird durch die Austrittsöffnung an dem Kopf der Flüssigkeitsstrahlsonde 61 Flüssigkeit in die Magenwand 82 so eingebracht, dass sich ein Flüssigkeitskissen vorteilhaft unter der gewünschten Ablationsstelle 81 bildet. Durch die Gaszuführungsleitungen 35a,b strömt Gas, beispielsweise Argon. Es werden nun der Generator 77 und die Schalteranordnung 36 aktiviert, so dass die Elektroden 41a,b abwechselnd zünden und einen Funken zur Mucosa 84 überspringen lassen. Der einhüllende Argonstrahl bildet vor jeder Elektrode 41a,b einen Plasmastrahl 83a,b, der in Figur 5 dargestellt ist. Die Plasmastrahlen 83a,b können sich dabei zu einem fächerförmigen Strahl vereinigen. Die Plasmastrahlen 83a,b treffen zeitlich versetzt nebeneinander auf der Mucosa 84 auf und koagulieren deren oberste Schicht, insbesondere deren Epithel 85 sowie der Lamina Propria 86 und Teile der Submucosa 87. Die Muscularis Propria 88 wird jedoch durch zuvor gebildete das Flüssigkeitskissen vorzugsweise verschont.

Durch das abwechselnde Ansteuern der beiden Plasmasonden mit beispielsweise 5 Hz kommt es makroskopisch zur Vereinigung der Plasmastrahlen 83a,b. So wird ein breiter Gewebestreifen mit einheitlicher Wirktiefe koaguliert. Die Breite des Gewebestreifens kann durch die Winkelanordnung der Enden 36a,b der Gaszuführungsleitungen 35a,b und der Elektroden 41a,b über 10 mm und im Einzelfall etwa 14 mm betragen. Die Behandlung nimmt ihren Fortgang, indem der Anwender mittels der Bedienelemente 14 des Endoskops 10 und durch entsprechende Führung des Bewegungsübertragungselements 25 das Instrumentenende 21 entlang eines Weges, insbesondere quer zu einer die Elektroden 41a,b verbindenden, gedachten Linie, in einem Abstand zu dem Gewebe über die Mucosa 84 führt, wobei er einen koagulierten Streifen Gewebes von etwa 12 mm bis 20 mm Breite hinterlässt. Auf diese Weise kann die Mucosa 84 in großer Zuverlässigkeit mit reduzierter Gefahr der Muscularisschädigung koaguliert werden.

Um das Einhalten eines bestimmten Mindestabstands zwischen den distalen Enden der Plasmasonden 44a,b und dem Gewebe beim Führen des Instrumentenendes 21 über das Gewebe in einem Winkel zwischen der Längsachse des Instrumentenendes 21 und dem Gewebe größer 0° und kleiner 90° (spitzer Winkel) zu erleichtern, kann das Instrument 11 ein Gleitelement 90 aufweisen, das derart an dem Instrument befestigt positioniert ist, dass das Instrument 11 über das Gleitelement 90 auf dem Gewebe 80 aufliegt. Figur 9 zeigt, stark schematisiert, ein Ausführungsbeispiel des Systems 91, das ein beispielsweise entsprechend der vorstehenden Beschreibung ausgebildetes und an einem Endoskop 10 gehaltenes Instrument 11 aufweist, das, wie mit einer gestrichelten Linie angedeutet, in einem spitzen Winkel β über das Gewebe 80 geführt wird. Das Gleitelement 90 des Instruments 11 kann, wie dargestellt, an dem Verbindungselement 29 oder beispielsweise an den distalen Enden der Plasmasonden 44a,b befestigt sein. Das Gleitelement 90 ist vorzugsweise derart an dem Instrument 11 angeordnet, dass das Instrument 11 über das Gleitelement 90 vorzugsweise auf dem Gewebe 80 proximal vor der Behandlungsstelle aufliegt. Beim Vor- und Zurückbewegen des Instruments 11 gleitet das Gleitelement 90 auf dem Gewebe, wobei, wenn ein maximaler Winkel β zwischen der Längsachse des distalen Instrumentenendes 21 und dem Gewebe 80 nicht überschritten wird, die Enden der Plasmasonden 44a,b mit einem bestimmten Mindestabstand a, oder wenn ein bestimmter Winkel zwischen Instrumentenende und Gewebe eingehalten wird, in einem bestimmten Abstand a über das Gewebe geführt werden. Das Gleitelement 90 fördert damit die Homogenität der Ablation. Der Winkel β zwischen der Längsachse des distalen Instrumentenendes 21 und dem Gewebe beträgt beim Führen über das Gewebe vorzugsweise zwischen mehr als 0° und 80°, besonders bevorzugt mindestens 20° bis maximal 30°. Der Abstand a bzw. der Mindestabstand a (in Figur 9 mit gestrichelter Linie angedeutet) zwischen den distalen Enden der Plasmasonden 44a,b einerseits und dem Gewebe 80 andererseits beim Führen des Instrumentenendes 21 über das Gewebe 80 mit auf dem Gewebe 80 aufliegendem Gleitelement 90 beträgt vorzugsweise mehr als 0 mm bis vorzugsweise maximal 10 mm, besonders bevorzugt mehr als 0 mm bis maximal 5 mm, beispielsweise mehr als 0 mm bis maximal 3 mm.

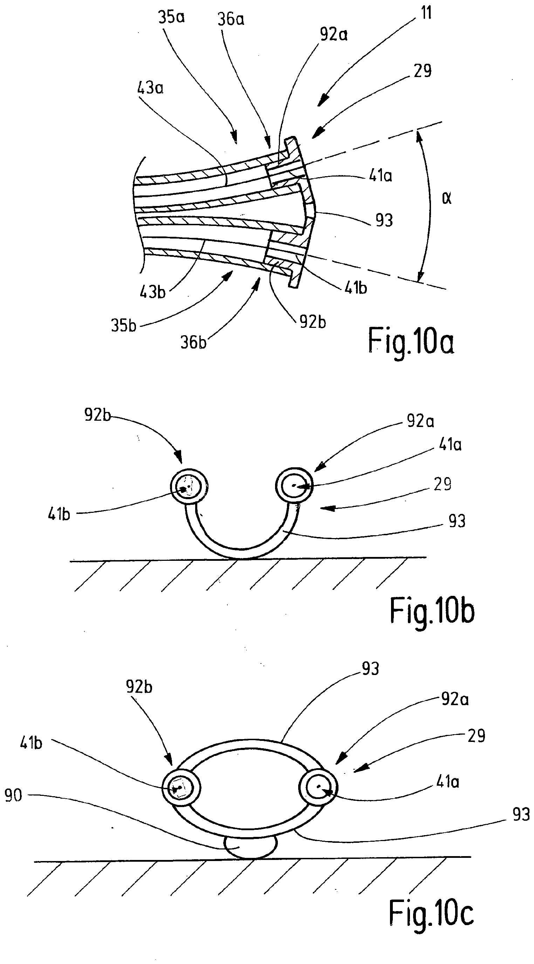

Figur 10a zeigt eine beispielhafte Ausführungsform des Instruments 11 mit einem Verbindungselement 29, das zwei Keramikrohrabschnitte 92a,b aufweist, die in den distalen Enden 36a,b der Gaszuführungsleitungen 35a,b stecken. Die Keramikrohrabschnitte 92a,b sind durch einen Verbindungsbügel 93 miteinander verbunden und von dem Verbindungsbügel 93 in dem spitzen Winkel α zueinander ausgerichtet, so dass die Enden 36a,b der Gaszuführungsleitungen 35a,b in dem spitzen Winkel α ausgerichtet sind. Die Elektroden 41a,b und die elektrischen Leitungen 43a,b sind durch eine durchgezogene Linie innerhalb der Gaszuführungsleitungen 35a,b veranschaulicht. Wie Figur 10b zeigt verläuft der Bügel 93 bogenförmig quer zur Längsachse des Instruments 11 unter den Keramikrohrabschnitten 92a,b. Der Verbindungsbügel 93 dient gleichzeitig als Gleitelement 90 für das Instrument 11.

Figur 10c zeigt eine Abwandlung des Ausführungsbeispiels des Instruments 11 gemäß Figur 10b mit einem weiteren Verbindungsbügel 93, der die Keramikrohrabschnitte verbindet und bogenförmig quer zur Längsachse über den Keramikrohrabschnitten 92a,b verläuft. An zumindest einem Bügel 93 ist ein Gleitschuh 90 angeordnet, der als Gleitelement dient.

Das Verbindungselement 29 einer der hierin beschriebenen Ausführungsformen kann einteilig oder mehrteilig sein. Zu Zusammenbau des Instruments 11 können die Plasmasonden 44a,b und/oder die Gaszuführungsleitungen 35a,b in die Sitze 51a,b beispielsweise eingeschoben, insbesondere bei einem einteiligen Verbindungselement 29, oder eingelegt oder eingedrückt, z.B. in eine Halbschale des Verbindungselements 29, werden. Das Verbindungselement 29 kann derart ausgebildet sein, dass gesondert voneinander verwendbare Plasmasonden 44a,b (Einzelsonden) jeweils an einem Verbindungselementteil befestigt sind, wobei die Verbindungselementteile, wenn Bedarf an einem erfindungsgemäßen Instrument 11 besteht, aneinander befestigt werden und die Einzelsonden 44a,b damit zu dem Instrument 11 mit dem Winkel α und dem Freiraum 45 zwischen den distalen Enden der Plasmasonden 44a,b angeordnet werden.

Erfindungsgemäß wird ein Instrument 11 bereitgestellt, das insbesondere zur großflächigen Gewebebehandlung, in einem Beispiel zur Ablation der Mucosa 84, verwendet werden kann, wobei das Instrument 11 wenigstens eine erste Gaszuführungsleitung 35a und wenigstens eine zweite Gaszuführungsleitung 35b aufweist, deren distale Enden 36a,b derart angeordnet sind, dass diese einen spitzen Winkel α einschließen, so dass die Enden 36a,b der Gaszuführungsleitungen 35a,b distal divergieren, um bei Beaufschlagung der Gaszuführungsleitungen 35a,b mit Gas distal divergierende Gasströmungen in den distalen Enden 36a,b der Gaszuführungsleitungen 35a,b zu erzeugen. Zwischen den distalen Enden 36a,b der Gaszuführungsleitungen 35a,b ist ein Freiraum 45 angeordnet, der es dem Benutzer des Instruments 11 erlaubt durch den Freiraum 45 zwischen den distalen Enden 36a,b der Gaszuführungsleitungen 35a,b hindurch auf den Gewebebereich 47 hinter dem distalen Ende 21 des Instruments 11 zu blicken. Der Freiraum 45 ist in Richtung distal vorzugsweise offen. Auf Grund des spitzen Winkels α kann mittels wenigstens teilweise in den Enden 36a,b der Gaszuführungsleitungen 35a,b angeordneten Elektroden 41a,b ein breiter Plasmastrahl erzeugt werden, mit dem die Mucosaablation durchgeführt werden kann. Dabei kann das Instrument 11 auf Grund der Sicht durch den Freiraum 45 besonders genau geführt werden. Zur Anordnung der Enden 36a,b der Gaszuführungsleitungen 35a,b wird ein Verbindungselement 29 bereitgestellt. Das Verbindungselement 29 kann an seinem distalen Ende 31 freistehende Fortsätze 53a,b aufweisen, durch die sich die Gaszuführungsleitungen 35a,b erstrecken. Alternativ oder zusätzlich sind die distalen Enden 36a,b der Gaszuführungsleitungen 36a,b vorzugsweise freistehend, sich von dem Verbindungselement 29 wegerstreckend angeordnet.

**Bezugszeichen:**

| | |
|---|---|
| 10 | Endoskop |
| 11 | Instrument |
| 12 | Magen |
| 13 | Speiseröhre |
| 14 | Bedienelemente |
| 15 | distales Ende des Endoskops |
| 16 | Kanäle |
| 17 | Mittel zur Bildübertragung |
| 18 | Schlauchhülle |
| 19a,b | Erstes Lumen, zweites Lumen |
| 20 | Ummantelungsschlauch |
| 21 | distales Instrumentenende/ Instrumentenkopf |
| 22 | Axialrichtung |
| 23 | Längsachse des Instrumentenendes |
| 24 | Fassung |
| 25 | Bewegungsübertragungselement |
| | |
| 27a,b | Gaszuführungsleitungsschläuche |
| 28 | Flüssigkeitszuführungsleitung |
| 29 | Verbindungselement |
| 30 | proximales Ende des Verbindungselements |
| 31 | distales Ende des Verbindungselements |
| 32 | Verdrehsicherung |
| 33 | Formnase, Feder |
| 34 | Nut, Schlitz |
| 35a,b | erste, zweite Gaszuführungsleitungen |
| 36a,b | Enden der Gaszuführungsleitung |
| 37a,b | Mittelachsen |
| 38a,b | Kunststoffleitung |
| 39a,b | Keramikrohr |
| 40a,b | distale Enden der Kunststoffleitungen |
| 41a,b | erste, zweite Elektroden |
| 43a,b | Elektrische Leitungen |
| 44a,b | Plasmasonden |
| 45 | Freiraum |
| 46a,b | Proximale Enden der Keramikrohre |
| 47 | Gewebebereich |
| 51a,b | Sitze |
| 53a,b | Fortsätze |
| 56a,b | Abschnitte der Gaszuführungsleitungen |
| 60 | Kopf einer weiteren Sonde |
| 61 | weitere Sonde/Flüssigkeitsstrahlsonde |
| 62 | Doppelpfeil |
| 65 | Sitz für die weitere Sonde |
| 66 | Mittelachse der weiteren Sonde |
| 68 | Pfeil |
| 69 | Pfeil |
| 75 | Schalteranordnung |
| 76 | Quelle |
| 77 | Hochfrequenzgenerator |
| 78 | Neutralelektrode |
| 80 | Gewebebereich |
| 81 | Gewebestelle |
| 82 | Magenwand |
| 83a,b | Plasmastrahl |
| 84 | Mukosa |
| 85 | Epithel |
| 86 | Lamina Propria |
| 87 | Submucosa |
| 88 | Muscularis Propria |
| 90 | Gleitelement/Gleitschuh |
| 91 | System |
| 92a,b | Keramikrohrabschnitte |
| 93 | Verbindungsbügel |
| α | spitzer Winkel |
| β | Winkel |
| a | Abstand |

## Patentansprüche

1. Instrument (11) zur Gewebebehandlung, insbesondere zur Mucosaablation, mit
einer ersten Gaszuführungsleitung (35a) mit einer wenigstens teilweise darin angeordneten ersten Elektrode (41a) und
einer zweiten Gaszuführungsleitung (35b) mit einer wenigstens teilweise darin angeordneten zweiten Elektrode (41b),
**dadurch gekennzeichnet, dass** die erste Gaszuführungsleitung (35a) und die zweite Gaszuführungsleitung (35b) zueinander derart angeordnet sind, dass das distale Ende (36a) der ersten Gaszuführungsleitung (35a) mit dem distalen Ende (36b) der zweiten Gaszuführungsleitung (35b) einen spitzen Winkel (α) einschließt, so dass die Gaszuführungsleitungen (35a,b) an ihren distalen Enden (36a,b) in distaler Richtung divergieren,
wobei zwischen dem distalen Ende (36a) der ersten Gaszuführungsleitung (35a) und dem distalen Ende (36b) der zweiten Gaszuführungsleitung (35b) ein Freiraum (45) angeordnet ist, der einen Blick zwischen den Enden (36a,b) der Gaszuführungsleitungen (35a,b) hindurch auf den Einwirkungsbereich des Instruments (11) vor den distalen Enden (36a,b) der Gaszuführungsleitungen (35a,b) gestattet.

2. Instrument (11) nach Anspruch 1, wobei die erste Gaszuführungsleitung (35a) und die erste Elektrode (41a) zu einer ersten Sonde (44a) und die zweite Gaszuführungsleitung (35b) und die zweite Elektrode (41b) zu einer zweiten Sonde (44b) gehören, wobei die erste Sonde (44a) und die zweite Sonde (44b) mit einem Verbindungselement (29) derart gehalten sind, dass das distale Ende (36a) der ersten Gaszuführungsleitung (35a) mit dem distalen Ende (36b) der zweiten Gaszuführungsleitung (35b) den spitzen Winkel (α) einschließt.

3. Instrument (11) nach Anspruch 2, wobei wenigstens eine der Sonden (44a,b) mit dem Verbindungselement (29) auswechselbar befestigt ist.

4. Instrument (11) nach einem der Ansprüche 2 bis 3, wobei die Enden (36a,b) der Gaszuführungsleitungen (35a,b) distal über das Verbindungselement (29) überstehen.

5. Instrument (11) nach einem der Ansprüche 2 bis 4, wobei die erste Gaszuführungsleitung (35a) in wenigstens einem Abschnitt durch eine Kunststoffleitung (38a) gebildet ist, die sich unterbrechungsfrei durch das Verbindungselement (29) erstreckt und wobei die zweite Gaszuführungsleitung (35b) in wenigstens einem Abschnitt durch eine Kunststoffleitung (38b) gebildet ist, die sich unterbrechungsfrei durch das Verbindungselement (29) erstreckt.

6. Instrument (11) nach einem der Ansprüche 1 bis 4, wobei die erste Gaszuführungsleitung (35a) ein Keramikrohr (39a) aufweist, dass in dem distalen Ende (40a) einer Kunststoffleitung (38a) der ersten Gaszuführungsleitung (35a) steckt und wobei die zweite Gaszuführungsleitung (35b) ein Keramikrohr (39b) aufweist, das in dem distalen Ende (40b) einer Kunststoffleitung (38b) der zweiten Gaszuführungsleitung (35b) steckt.

7. Instrument (11) nach Anspruch 6, wenn abhängig von einem der Ansprüche 2 bis 4, wobei die Keramikrohre (39a,b) mit ihren proximalen Enden (46a,b) in dem Verbindungselement (29) angeordnet sind.

8. Instrument nach Anspruch 6 oder 7, wobei die Keramikrohre (39a,b) an den Enden (36a,b) der Gaszuführungsleitungen (35a,b) distal über die Kunststoffleitungen (38a,b) überstehen.

9. Instrument (11) nach einem der vorhergehenden Ansprüche, wobei das Instrument (11) eine weitere Sonde (61), beispielsweise eine Flüssigkeitsstrahlsonde, aufweist, deren distales Ende (60) an dem distalen Instrumentenende (21) angeordnet ist.

10. Instrument (11) nach Anspruch 9, wenn zumindest abhängig von Anspruch 2, wobei die weitere Sonde (61) in einem Sitz (65) des Verbindungselements (29) für die weitere Sonde (61) angeordnet ist.

11. Instrument (11) nach Anspruch 9 oder Anspruch 10, wobei die weitere Sonde (61) relativ zu der Anordnung des Endes (36a) der ersten Gaszuführungseinrichtung (35a) und des Endes (36b) der zweiten Gaszuführungseinrichtung (35b) in Richtung proximal aus dem Freiraum (45) heraus bewegbar ist und/oder wobei die weitere Sonde (61) über die distalen Enden (36a,b) der Gaszuführungseinrichtungen (35a,b) hinaus in Richtung distal verschiebbar ist.

12. Instrument (11) nach einem der vorhergehenden Ansprüche, wobei das Instrument (11) ein Gleitelement (90) zum Führen des Instruments (11) über das Gewebe aufweist.

13. Instrument (11) nach einem der vorhergehenden Ansprüche, wenn zumindest abhängig von Anspruch 2, wobei das Verbindungselement (29) distale Fortsätze (53a,b) aufweist, wobei sich die erste Gaszuführungsleitung (35a) durch den ersten Fortsatz (53a) und sich die zweite Gaszuführungsleitung (35b) durch den zweiten Fortsatz (53b) erstreckt, wobei sich der Freiraum (45) zwischen die Fortsätze (53a,b) erstreckt.

## Claims

1. Instrument (11) for tissue treatment, in particular for mucosa ablation, with
a first gas supply line (35a) with a first electrode (41a) arranged at least partially therein, and
a second gas supply line (35b) with a second electrode (41b) arranged at least partially therein,
**characterised in that**
the first gas supply line (35a) and the second gas supply line (35b) are arranged relative to each other such that the distal end (36a) of the first gas supply line 35a) encloses an acute angle (a) with the distal end (36b) of the second gas supply line (35b), so that the gas supply lines (35a,b) diverge in the distal direction at their distal ends (36a,b),
wherein a space (45) is arranged between the distal end (36a) of the first gas supply line (35a) and the distal end (36b) of the second gas supply line (35b), which space allows a view between the ends (36a,b) of the gas supply lines (35a,b) onto the action region of the instrument (11) in front of the distal ends (36a,b) of the gas supply lines (35a,b).

2. Instrument (11) according to claim 1, wherein the first gas supply line (35a) and the first electrode (41a) belong to a first probe (44a), and the second gas supply line (35b) and the second electrode (41b) belong to a second probe (44b), wherein the first probe (44a) and the second probe (44b) are held with a connecting element (29) such that the distal end (36a) of the first gas supply line (35a) encloses the acute angle (a) with the distal end (36b) of the second gas supply line (35b).

3. Instrument (11) according to claim 2, wherein at least one of the probes (44a,b) is attached to the connecting element (29) in exchangeable fashion.

4. Instrument (11) according to one of claims 2 to 3, wherein the ends (36a,b) of the gas supply lines (35a,b) protrude distally over the connecting element (29).

5. Instrument (11) according to any of claims 2 to 4, wherein at least one portion of the first gas supply line (35a) is formed by a plastic line (38a) which extends uninterruptedly through the connecting element (29), and wherein at least one portion of the second gas supply line (35b) is formed by a plastic line (38b) which extends uninterruptedly through the connecting element (29).

6. Instrument (11) according to any of claims 1 to 4, wherein the first gas supply line (35a) comprises a ceramic tube (39a) which is inserted in the distal end (40a) of a plastic line (38a) of the first gas supply line (35a), and wherein the second gas supply line (35b) comprises a ceramic tube (39b) which is inserted in the distal end (40b) of a plastic line (38b) of the second gas supply line (35b).

7. Instrument (11) according to claim 6 insofar as dependent on one of claims 2 to 4, wherein the proximal ends (46a,b) of the ceramic tubes (39a,b) are arranged in the connecting element (29).

8. Instrument (11) according to claim 6 or 7, wherein at the ends (36a,b) of the gas supply lines (35a,b), the ceramic tubes (39a,b) protrude distally over the plastic lines (38a,b).

9. Instrument (11) according to any of the preceding claims, wherein the instrument (11) comprises a further probe (61), for example a liquid jet probe, the distal end (60) of which is arranged at the distal instrument end (21).

10. Instrument (11) according to claim 9 insofar as dependent at least on claim 2, wherein the further probe (61) is arranged in a seat (65) for the further probe (61) on the connecting element (29).

11. Instrument (11) according to claim 9 or claim 10, wherein the further probe (61) can be moved in the proximal direction out of the space (45) relative to the arrangement of the end (36a) of the first gas supply device¹ (35a) and the end (36b) of the second gas supply device (35b), and/or wherein the further probe (61) can be moved in the distal direction beyond the distal ends (36a,b) of the gas supply devices (35a,b).
¹ *Translator's note: In this claim, the phrase "gas supply device" is used instead of "gas supply line".*

12. Instrument (11) according to any of the preceding claims, wherein the instrument (11) comprises a slide element (90) for guiding the instrument (11) over the tissue.

13. Instrument (11) according to any of the preceding claims insofar as dependent at least on claim 2, wherein the connecting element (29) comprises distal extensions (53a,b), wherein the first gas supply line (35a) extends through the first extension (53a), and the second gas supply line (35b) extends through the second extension (53b), wherein the space (45) extends between the extensions (53a,b).

## Revendications

1. Instrument (11) destiné au traitement des tissus, en particulier à l'ablation de muqueuse, comprenant
une première conduite d'alimentation en gaz (35a) comportant une première électrode (41a) disposée au moins partiellement dans celle-ci, et
une deuxième conduite d'alimentation en gaz (35b) comportant une deuxième électrode (41b) disposée au moins partiellement dans celle-ci,
**caractérisé en ce que**
la première conduite d'alimentation en gaz (35a) et la deuxième conduite d'alimentation en gaz (35b) sont disposées l'une par rapport à l'autre de manière telle que l'extrémité distale (36a) de la première conduite d'alimentation en gaz (35a) forme un angle aigu (a) avec l'extrémité distale (36b) de la deuxième conduite d'alimentation en gaz (35b), de sorte qu'à leurs extrémités distales (36a, b) les conduites d'alimentation en gaz (35a, b) divergent dans la direction distale,
sachant qu'il est prévu entre l'extrémité distale (36a) de la première conduite d'alimentation en gaz (35a) et l'extrémité distale (36b) de la deuxième conduite d'alimentation en gaz (35b), un espace libre (45) qui permet de regarder entre les extrémités (36a, b) des conduites d'alimentation en gaz (35a, b) pour voir la zone d'action de l'instrument (11) située devant les extrémités distales (36a, b) des conduites d'alimentation en gaz (35a, b).

2. Instrument (11) selon la revendication 1, dans lequel la première conduite d'alimentation en gaz (35a) et la première électrode (41a) font partie d'une première sonde (44a), et la deuxième conduite d'alimentation en gaz (35b) et la deuxième électrode (41b) font partie d'une deuxième sonde (44b), la première sonde (44a) et la deuxième sonde (44b) étant maintenues à l'aide d'un élément de liaison (29), de manière à ce que l'extrémité distale (36a) de la première conduite d'alimentation en gaz (35a) forme l'angle aigu (a) avec l'extrémité distale (36b) de la deuxième conduite d'alimentation en gaz (35b).

3. Instrument (11) selon la revendication 2, dans lequel au moins l'une des sondes (44a, b) est fixée de manière échangeable à l'élément de liaison (29).

4. Instrument (11) selon l'une des revendications 2 à 3, dans lequel les extrémités (36a, b) des conduites d'alimentation en gaz (35a, b) dépassent dans le sens distal par rapport à l'élément de liaison (29).

5. Instrument (11) selon l'une des revendications 2 à 4, dans lequel la première conduite d'alimentation en gaz (35a) est constituée, dans au moins une portion, d'une conduite en matière plastique (38a) qui s'étend sans interruption à travers l'élément de liaison (29), et dans lequel la deuxième conduite d'alimentation en gaz (35b) est constituée, dans au moins une portion, d'une conduite en matière plastique (38b) qui s'étend sans interruption à travers l'élément de liaison (29).

6. Instrument (11) selon l'une des revendications 1 à 4, dans lequel la première conduite d'alimentation en gaz (35a) présente un tube en céramique (39a) qui est inséré dans l'extrémité distale (40a) d'une conduite en matière plastique (38a) de la première conduite d'alimentation en gaz (35a), et dans lequel la deuxième conduite d'alimentation en gaz (35b) présente un tube en céramique (39b) qui est inséré dans l'extrémité distale (40b) d'une conduite en matière plastique (38a) de la deuxième conduite d'alimentation en gaz (35b).

7. Instrument (11) selon la revendication 6, si elle est dépendante des revendications 2 à 4, dans lequel les tubes en céramique (39a, b) sont disposés avec leurs extrémités proximales (46a, b) dans l'élément de liaison (29).

8. Instrument (11) selon la revendication 6 ou 7, dans lequel les tubes en céramique (39a, b) dépassent dans le sens distal par rapport aux conduites en matière plastique (38a, b), aux extrémités (36a, b) des conduites d'alimentation en gaz (35a, b).

9. Instrument (11) selon l'une des revendications précédentes, dans lequel l'instrument (11) présente une sonde supplémentaire (61), par exemple une sonde à jet de liquide, dont l'extrémité distale (60) est disposée sur l'extrémité d'instrument distale (21).

10. Instrument (11) selon la revendication 9, si elle est dépendante au moins de la revendication 2, dans lequel la sonde supplémentaire (61) est disposée dans un siège (65) de l'élément de liaison (29) destiné à la sonde supplémentaire (61).

11. Instrument (11) selon la revendication 9 ou la revendication 10, dans lequel la sonde supplémentaire (61) peut être déplacée hors de l'espace libre (45), dans la direction proximale, par rapport à la disposition de l'extrémité (36a) de la première conduite d'alimentation en gaz (35a) et de l'extrémité (36b) de la deuxième conduite d'alimentation en gaz (35b), et/ou dans lequel la sonde supplémentaire (61) peut être déplacée dans le sens distal, au-delà des extrémités distales (36a, b) des dispositifs d'alimentation en gaz (35a, b).

12. Instrument (11) selon l'une des revendications précédentes, dans lequel l'instrument (11) présente un élément de glissement (90) pour guider l'instrument (11) sur les tissus.

13. Instrument (11) selon l'une des revendications précédentes, si elle est au moins dépendante de la revendication 2, dans lequel l'élément de liaison (29) présente des prolongements distaux (53a, b) et la première conduite d'alimentation en gaz (35a) s'étend dans le premier prolongement (53a) et la deuxième conduite d'alimentation en gaz (35b) s'étend dans le deuxième prolongement (53b), l'espace libre (45) s'étendant entre les prolongements (53a, b).
